# EUROPEAN PATENT APPLICATION

(11) **EP 1 783 219 A1**
(43) Date of publication of application: **09.05.2007**
(21) Application number: 05758233.0
(22) Date of filing: 08.07.2005
(51) Int. Cl.: C12N 15/09, C07K 14/47, C12N 1/15, C12N 1/19, C12N 1/21, C12N 5/10, C12P 21/02, C12Q 1/02, G01N 33/15, G01N 33/50

(54) **METHOD OF SEARCHING FOR NOVEL DRUG DISCOVERY TARGETS**

(30) Priority: 09.07.2004 JP 2004203672
(71) Applicant: Genofunction, Inc., Tsukuba-shi, Ibaraki 305-0856 (JP)
(72) Inventor: GOTO, Rika, 3050856 (JP); SUZUKI, Yosuke, 3050856 (JP)
(74) Representative: Grünecker, Kinkeldey, Stockmair & Schwanhäusser Anwaltssozietät
(86) International application number: PCT/JP2005/012655
(87) International publication number: WO 2006/006520

(57) **Abstract**

The present inventors conducted dedicated studies to solve the above objectives. As a result, the inventors developed libraries of expression vectors for stem-loop RNA molecules that allow comprehensive screening for gene function, and methods of comprehensively screening for drug target molecules using these libraries. By using these methods, the inventors identified drug target molecules whose suppressed expression is expected to have heightened effects.

SEQUENCE LISTING

<110> GENOFUNCTION, INC.

<120> METHOD OF SEARCHING FOR NOVEL TARGET OF DRUG DISCOVERY

<130> GFU-A0401P

<140> PCT/JP2005/012655
<141> 2005-07-08

<150> JP 2004-203672
<151> 2004-07-09

<160> 226

<170> PatentIn Ver. 2.0

<210> 1
<211> 28
<212> DNA
<213> Homo sapiens

<400> 1
ggaggagtcg ggcgccgagg agtccggc                                    28

<210> 2
<211> 28
<212> DNA
<213> Homo sapiens

<400> 2
gacggacagc ctatttttgc caatatca                                    28

<210> 3
<211> 25

<212> DNA
<213> Homo sapiens

<400> 3
tgccccggag ggcggagaac tggga          25

<210> 4
<211> 28
<212> DNA
<213> Homo sapiens

<400> 4
tggagcggct gacacaggag cgcattgc          28

<210> 5
<211> 25
<212> DNA
<213> Homo sapiens

<400> 5
   ccgaggagtc cggcccggaa gagtc          25

<210> 6
<211> 28
<212> DNA
<213> Homo sapiens

<400> 6
tgggagggct cgcgcgagtt caccgagc          28

<210> 7
<211> 29
<212> DNA
<213> Homo sapiens

<400> 7

tgtatactct gaaagagcga tgcctccag          29

<210> 8
<211> 28
<212> DNA
<213> Homo sapiens

<400> 8
gctccaggtc tttctgcaca tttgggtg          28

<210> 9
<211> 27
<212> DNA
<213> Homo sapiens

<400> 9
gtgcaatgcg ctcctgtgtc ggtcgat          27

<210> 10
<211> 27
<212> DNA
<213> Homo sapiens

<400> 10
cccgactcct ccccgccgtc ctcttca          27

<210> 11
<211> 25
<212> DNA
<213> Homo sapiens

<400> 11
tcaccgagcg cagcacgggc cgcgg          25

<210> 12
<211> 27

<212> DNA
<213> Homo sapiens

<400> 12
ccaggcggca gcgttgggta gggctgc          27

<210> 13
<211> 25
<212> DNA
<213> Homo sapiens

<400> 13
cctgcctcct ccgcgcctac ctcgg          25

<210> 14
<211> 29
<212> DNA
<213> Homo sapiens

<400> 14
gaggagtccg gccccggaag agtccggcc          29

<210> 15
<211> 28
<212> DNA
<213> Homo sapiens

<400> 15
   tccgggccgg actcctcggc gcccgact          28

<210> 16
<211> 34
<212> DNA
<213> Homo sapiens

<400> 16

cctgcctcct ccgcgcctac ctcggcctcg tccc          34

<210> 17
<211> 31
<212> DNA
<213> Homo sapiens

<400> 17
tgaactcgcg cgagccctcc cacatctccc c          31

<210> 18
<211> 29
<212> DNA
<213> Homo sapiens

<400> 18
cctggagcgg ttgacacagg agcgcattg          29

<210> 19
<211> 28
<212> DNA
<213> Homo sapiens

<400> 19
gaggagtccg gcccggaaga gtccggcc          28

<210> 20
<211> 35
<212> DNA
<213> Homo sapiens

<400> 20
tgaactcgcg cgagccctcc caggtcatct tctgc          35

<210> 21
<211> 28

<212> DNA
<213> Homo sapiens

<400> 21
tcagggccgt actcttcgac gcctgcct          28

<210> 22
<211> 31
<212> DNA
<213> Homo sapiens

<400> 22
cccaggtcat cttctgcaat cgcagtccgc g          31

<210> 23
<211> 24
<212> DNA
<213> Homo sapiens

<400> 23
ctctgaaaga gcgatgcctc cagg          24

<210> 24
<211> 24
<212> DNA
<213> Homo sapiens

<400> 24
ccaggtcttt ctgcacattt gggt          24

<210> 25
<211> 24
<212> DNA
<213> Homo sapiens

<400> 25

gaagagccaa aggtgacctc ggta 24

<210> 26
<211> 26
<212> DNA
<213> Homo sapiens

<400> 26
caatgttgac ggacagccta tttttg 26

<210> 27
<211> 27
<212> DNA
<213> Homo sapiens

<400> 27
gcccatcgtg tgtccctgcg tctctga 27

<210> 28
<211> 25
<212> DNA
<213> Homo sapiens

<400> 28
cacagctacc gaggtcacct ttggc 25

<210> 29
<211> 33
<212> DNA
<213> Homo sapiens

<400> 29
tgcatctctg aagagccaaa ggtgacctcg gta 33

<210> 30
<211> 25

<212> DNA
<213> Homo sapiens

<400> 30
tcacctttgg ctcttcagag atgca 25

<210> 31
<211> 24
<212> DNA
<213> Homo sapiens

<400> 31
gggacgaggc cgaggtaggc gcgg 24

<210> 32
<211> 24
<212> DNA
<213> Homo sapiens

<400> 32
cctggcacgg gccgccgcat ccac 24

<210> 33
<211> 30
<212> DNA
<213> Homo sapiens

<400> 33
tcttcagggc cgtactcttc gacgcctgcc 30

<210> 34
<211> 26
<212> DNA
<213> Homo sapiens

<400> 34

tggatatatc taagcccttc tgcaca 26

<210> 35
<211> 24
<212> DNA
<213> Homo sapiens

<400> 35
tgttttcttc ccttatctta ctgt 24

<210> 36
<211> 24
<212> DNA
<213> Homo sapiens

<400> 36
cagttggtca gccatgctgc aagc 24

<210> 37
<211> 25
<212> DNA
<213> Homo sapiens

<400> 37
gcctaaaaat ccattccaag ttgta 25

<210> 38
<211> 30
<212> DNA
<213> Homo sapiens

<400> 38
acaacatgct gacagttcct aaagaaaact 30

<210> 39
<211> 29

<212> DNA
<213> Homo sapiens

<400> 39
tccctgaatt tctgacaatg atgtgcttt          29

<210> 40
<211> 25
<212> DNA
<213> Homo sapiens

<400> 40
tgaccaattg actgaagagc agatt          25

<210> 41
<211> 27
<212> DNA
<213> Homo sapiens

<400> 41
gagacacagc agcgctgggc ctactgc          27

<210> 42
<211> 26
<212> DNA
<213> Homo sapiens

<400> 42
gaacacacac tccacccatg ccgagg          26

<210> 43
<211> 29
<212> DNA
<213> Homo sapiens

<400> 43

ctccttaatc ccgtccactg ggttatgga 29

<210> 44
<211> 30
<212> DNA
<213> Homo sapiens

<400> 44
cttccagggc tgagataccg ctggtccaca 30

<210> 45
<211> 29
<212> DNA
<213> Homo sapiens

<400> 45
tgtaggttgg tacttccccc attattcct 29

<210> 46
<211> 31
<212> DNA
<213> Homo sapiens

<400> 46
aatcttcaat actctttaaa agcccacttc c 31

<210> 47
<211> 31
<212> DNA
<213> Homo sapiens

<400> 47
tgagctgaag gaaaagattg atcgccgttc t 31

<210> 48
<211> 27

<212> DNA
<213> Homo sapiens

<400> 48
taatcagtgg tggaagaacg gtctcag          27

<210> 49
<211> 30
<212> DNA
<213> Homo sapiens

<400> 49
tgatggccac tagtggtgga cttgcccgaa          30

<210> 50
<211> 32
<212> DNA
<213> Homo sapiens

<400> 50
tgcagcatca ccagacttca agaatttagg gc          32

<210> 51
<211> 28
<212> DNA
<213> Homo sapiens

<400> 51
ggttgcagtt aactgagatt gcgccact          28

<210> 52
<211> 30
<212> DNA
<213> Homo sapiens

<400> 52

gcaggcgcgc ctggccgcga acccggaccc 30

<210> 53
<211> 26
<212> DNA
<213> Homo sapiens

<400> 53
agcaccctgt tgtggctcta taagca 26

<210> 54
<211> 24
<212> DNA
<213> Homo sapiens

<400> 54
ggctgggatt acaggtgtga gcca 24

<210> 55
<211> 26
<212> DNA
<213> Homo sapiens

<400> 55
ggccccagct aactgtaccg ttagca 26

<210> 56
<211> 24
<212> DNA
<213> Homo sapiens

<400> 56
cccaaagggg ccatctcctg tatg 24

<210> 57
<211> 25

<212> DNA
<213> Homo sapiens

<400> 57
cgatgaggtt tttcacctgt gtgga 25

<210> 58
<211> 27
<212> DNA
<213> Homo sapiens

<400> 58
agaagcccct gcgctggctg cctcata 27

<210> 59
<211> 26
<212> DNA
<213> Homo sapiens

<400> 59
ccatgaagaa gcttcatctc aacctc 26

<210> 60
<211> 28
<212> DNA
<213> Homo sapiens

<400> 60
tgagatgagg aatgaaaaca tcagggtg 28

<210> 61
<211> 27
<212> DNA
<213> Homo sapiens

<400> 61

gaatgaaaac ttgccagttt attctgg          27

<210> 62
<211> 25
<212> DNA
<213> Homo sapiens

<400> 62
tctcaaaacc tcatggcagg ctcca          25

<210> 63
<211> 32
<212> DNA
<213> Homo sapiens

<400> 63
cttctttctc tttggtccct cgcccttgta cc          32

<210> 64
<211> 27
<212> DNA
<213> Homo sapiens

<400> 64
tatgggctct acattgtcag ctacttg          27

<210> 65
<211> 27
<212> DNA
<213> Homo sapiens

<400> 65
gacggattct gttgacactg gtgatca          27

<210> 66
<211> 30

<212> DNA
<213> Homo sapiens

<400> 66
tggcttgtta gaaggctgaa agatgatggc                    30

<210> 67
<211> 26
<212> DNA
<213> Homo sapiens

<400> 67
tgggaacagt gtctcctgac ttgtca                26

<210> 68
<211> 31
<212> DNA
<213> Homo sapiens

<400> 68
cctcgtactt ctcctaccac tgctgcccag a                    31

<210> 69
<211> 29
<212> DNA
<213> Homo sapiens

<400> 69
ttaatagtca cgtttttgcc ccttctatt                29

<210> 70
<211> 27
<212> DNA
<213> Homo sapiens

<400> 70

gttatgcctc actggtctac cttgttc          27

<210> 71
<211> 29
<212> DNA
<213> Homo sapiens

<400> 71
ctattgccta cgtccctgtc gcccaggtc          29

<210> 72
<211> 20
<212> DNA
<213> Homo sapiens

<400> 72
ccacccttct ttgagacacc          20

<210> 73
<211> 25
<212> DNA
<213> Homo sapiens

<400> 73
agtgtttcaa gaaggcatct ttacc          25

<210> 74
<211> 27
<212> DNA
<213> Homo sapiens

<400> 74
ccatgttttc ttgaggtgcg gcttagt          27

<210> 75
<211> 25

<212> DNA
<213> Homo sapiens

<400> 75
actgaggctg aatccgttat taact                25

<210> 76
<211> 26
<212> DNA
<213> Homo sapiens

<400> 76
ttgagaccat cctggccagc atggtg               26

<210> 77
<211> 27
<212> DNA
<213> Homo sapiens

<400> 77
actgagtgaa agagggggct cccctca              27

<210> 78
<211> 29
<212> DNA
<213> Homo sapiens

<400> 78
catatgcctc cgctacttgc ttgaatttt            29

<210> 79
<211> 26
<212> DNA
<213> Homo sapiens

<400> 79

agggattctc ccgcctcagc ctcctg 26

<210> 80
<211> 24
<212> DNA
<213> Homo sapiens

<400> 80
tgctgtgtgg gtgcggccac gtat 24

<210> 81
<211> 29
<212> DNA
<213> Homo sapiens

<400> 81
ggtgaataga ggacaagcga caggcagtg 29

<210> 82
<211> 29
<212> DNA
<213> Homo sapiens

<400> 82
tccagggcca tgtctgactt ctgaatggc 29

<210> 83
<211> 25
<212> DNA
<213> Homo sapiens

<400> 83
cttactcacc accgtttatc cagtg 25

<210> 84
<211> 28

<212> DNA
<213> Homo sapiens

<400> 84
tcccgcgaag cccaatcgcg aggccgcg          28

<210> 85
<211> 26
<212> DNA
<213> Homo sapiens

<400> 85
gctccagggt ggtggagact gttttc          26

<210> 86
<211> 27
<212> DNA
<213> Homo sapiens

<400> 86
agttcgagac cagcctgatc agcatgg          27

<210> 87
<211> 29
<212> DNA
<213> Homo sapiens

<400> 87
ccgctcgata gtttcagaaa tggtgcgtt          29

<210> 88
<211> 30
<212> DNA
<213> Homo sapiens

<400> 88

cccagttcct ctggggctct cagaatcact 30

<210> 89
<211> 26
<212> DNA
<213> Homo sapiens

<400> 89
tgggaggctg aggcaggaaa accgcc 26

<210> 90
<211> 27
<212> DNA
<213> Homo sapiens

<400> 90
ggttggtgtc agtgattttt gtgagac 27

<210> 91
<211> 26
<212> DNA
<213> Homo sapiens

<400> 91
cgccaggcaa agcaggagtc cactga 26

<210> 92
<211> 27
<212> DNA
<213> Homo sapiens

<400> 92
ccgccgcccg tctcctgtcg cgcagct 27

<210> 93
<211> 27

<212> DNA
<213> Homo sapiens

<400> 93
gcattcacca gaaccagcct tgtctat          27

<210> 94
<211> 26
<212> DNA
<213> Homo sapiens

<400> 94
tgatggcggc ggagtgagaa gttaat          26

<210> 95
<211> 31
<212> DNA
<213> Homo sapiens

<400> 95
cagagggaag tccaagccca tggggttctg g          31

<210> 96
<211> 29
<212> DNA
<213> Homo sapiens

<400> 96
acactaacat atacgcccca ttccctgct          29

<210> 97
<211> 27
<212> DNA
<213> Homo sapiens

<400> 97

tgagtagctg ggactacagg tgcgcac 27

<210> 98
<211> 28
<212> DNA
<213> Homo sapiens

<400> 98
tatacagagc ttcctcacct gttcgggc 28

<210> 99
<211> 28
<212> DNA
<213> Homo sapiens

<400> 99
tctgggtact tggtcagtgt ttgtttgc 28

<210> 100
<211> 30
<212> DNA
<213> Homo sapiens

<400> 100
tgattttgtt tttagcttac tgctgccacc 30

<210> 101
<211> 26
<212> DNA
<213> Homo sapiens

<400> 101
tgaatacatg attacccaca gcggca 26

<210> 102
<211> 27

<212> DNA
<213> Homo sapiens

<400> 102
acttctcctg tccctgggac gtgcgtc          27

<210> 103
<211> 25
<212> DNA
<213> Homo sapiens

<400> 103
tgaactcctg acctcgtgat ccacc          25

<210> 104
<211> 26
<212> DNA
<213> Homo sapiens

<400> 104
ctgggtgagt ttcggggatt gccata          26

<210> 105
<211> 26
<212> DNA
<213> Homo sapiens

<400> 105
tagggactgt tagctggagt ttatta          26

<210> 106
<211> 25
<212> DNA
<213> Homo sapiens

<400> 106

ccccggagcc cccaaaccgc agaca          25

<210> 107
<211> 27
<212> DNA
<213> Homo sapiens

<400> 107
ggctggagtg cagtggcacg atctcgg          27

<210> 108
<211> 28
<212> DNA
<213> Homo sapiens

<400> 108
tcccccctcc tctgaaacag ctgcctct          28

<210> 109
<211> 26
<212> DNA
<213> Homo sapiens

<400> 109
gggccacaaa gcctgctcca cacctt          26

<210> 110
<211> 26
<212> DNA
<213> Homo sapiens

<400> 110
cccaggacca tagccggaag acggag          26

<210> 111
<211> 29

<212> DNA
<213> Homo sapiens

<400> 111
tgtaaaaagc tctaggtgca caataagca          29

<210> 112
<211> 30
<212> DNA
<213> Homo sapiens

<400> 112
tctgggtaaa attactctgt actagtgtag                    30

<210> 113
<211> 24
<212> DNA
<213> Homo sapiens

<400> 113
cccagaacga caacgccata tttc               24

<210> 114
<211> 24
<212> DNA
<213> Homo sapiens

<400> 114
ccaagttcca cagctgcaag gcgt               24

<210> 115
<211> 24
<212> DNA
<213> Homo sapiens

<400> 115

cgatgttgaa gcccagcgtt gggg 24

<210> 116
<211> 25
<212> DNA
<213> Homo sapiens

<400> 116
ccctgggcac tggcctagag cctca 25

<210> 117
<211> 28
<212> DNA
<213> Homo sapiens

<400> 117
tggaaagaac catgtggaaa tggaatag 28

<210> 118
<211> 31
<212> DNA
<213> Homo sapiens

<400> 118
taaatgtggc tgatgatgca gttgcttagc t 31

<210> 119
<211> 26
<212> DNA
<213> Homo sapiens

<400> 119
ggcaatgtcg aatctatgag cttcga 26

<210> 120
<211> 30

<212> DNA
<213> Homo sapiens

<400> 120
gaaatagaag cccagagaga ggtaggtgta 30

<210> 121
<211> 27
<212> DNA
<213> Homo sapiens

<400> 121
aactgccaga gttttctctg ggtgtta 27

<210> 122
<211> 29
<212> DNA
<213> Homo sapiens

<400> 122
aacgcctccc ggctctcgtc agcctccca 29

<210> 123
<211> 28
<212> DNA
<213> Homo sapiens

<400> 123
actatctctt ttaactggaa ccaaacca 28

<210> 124
<211> 30
<212> DNA
<213> Homo sapiens

<400> 124

taatttgcct tcccctactc tcttcccatc                    30

<210> 125
<211> 26
<212> DNA
<213> Homo sapiens

<400> 125
caccgtgggt tgtttccatt gggaca                26

<210> 126
<211> 26
<212> DNA
<213> Homo sapiens

<400> 126
agaatggcgt cacgtagtcg gtgtgc                26

<210> 127
<211> 26
<212> DNA
<213> Homo sapiens

<400> 127
agcctccttc agcagctact gagcta                26

<210> 128
<211> 27
<212> DNA
<213> Homo sapiens

<400> 128
agagagcaga agccatgaac tgtaagg               27

<210> 129
<211> 26

<212> DNA
<213> Homo sapiens

<400> 129
ggtctcgaac tcctgacttc aagtga 26

<210> 130
<211> 28
<212> DNA
<213> Homo sapiens

<400> 130
ggtgagctgg atattccact cggggcct 28

<210> 131
<211> 27
<212> DNA
<213> Homo sapiens

<400> 131
taggctggtc tcaaactcct gggctca 27

<210> 132
<211> 25
<212> DNA
<213> Homo sapiens

<400> 132
aattaggtaa gattgggaca gaatt 25

<210> 133
<211> 26
<212> DNA
<213> Homo sapiens

<400> 133

tcctttagcg gtcattgcct tctgac 26

<210> 134
<211> 28
<212> DNA
<213> Homo sapiens

<400> 134
ggggatggtc tcgccgcctt ggcaggga 28

<210> 135
<211> 30
<212> DNA
<213> Homo sapiens

<400> 135
gccgagatca cgccactgca cttcagcctg 30

<210> 136
<211> 26
<212> DNA
<213> Homo sapiens

<400> 136
cccaatctta ccagatgcta ctggac 26

<210> 137
<211> 30
<212> DNA
<213> Homo sapiens

<400> 137
cggccgggca acatgctcct ctgcgttggt 30

<210> 138
<211> 31

<212> DNA
<213> Homo sapiens

<400> 138
tggggcgcgg tggctcacgc ctgtggtctc a          31

<210> 139
<211> 33
<212> DNA
<213> Homo sapiens

<400> 139
tatcctgaca atgttcctgt attttttctg gat          33

<210> 140
<211> 26
<212> DNA
<213> Homo sapiens

<400> 140
gccaaggaca ctgatgcctc cccttt          26

<210> 141
<211> 33
<212> DNA
<213> Homo sapiens

<400> 141
cccatgatat aggaggcatt gacgtcgcgg ccg          33

<210> 142
<211> 27
<212> DNA
<213> Homo sapiens

<400> 142

agttggcctt ggtccttttc tttcggg 27

<210> 143
<211> 27
<212> DNA
<213> Homo sapiens

<400> 143
ccccattgct ctgtgggcgc cccgccc 27

<210> 144
<211> 29
<212> DNA
<213> Homo sapiens

<400> 144
cacccgccat cacgcccggc taaggcttc 29

<210> 145
<211> 26
<212> DNA
<213> Homo sapiens

<400> 145
ccttcgtctc caacctccag gggcag 26

<210> 146
<211> 25
<212> DNA
<213> Homo sapiens

<400> 146
gcctgtgcac cggcagggga gaagg 25

<210> 147
<211> 28

<212> DNA
<213> Homo sapiens

<400> 147
ggttgcagtt ttaaattggg gaatttga          28

<210> 148
<211> 30
<212> DNA
<213> Homo sapiens

<400> 148
gaaatgaatg agcttctgac tttacaggat                    30

<210> 149
<211> 25
<212> DNA
<213> Homo sapiens

<400> 149
ggcgagagcc accgcaccca gcccc             25

<210> 150
<211> 30
<212> DNA
<213> Homo sapiens

<400> 150
agggaaaaca gagagagggt caggcctagt                    30

<210> 151
<211> 27
<212> DNA
<213> Homo sapiens

<400> 151

tggggtaatc agaggcagcg ctccaga          27

<210> 152
<211> 25
<212> DNA
<213> Homo sapiens

<400> 152
gccttaacct cccaaattgc tgcga          25

<210> 153
<211> 29
<212> DNA
<213> Homo sapiens

<400> 153
cctgaatcac agagtagtac ccttccttg          29

<210> 154
<211> 28
<212> DNA
<213> Homo sapiens

<400> 154
gtagattcac agtgctccga agatcggc          28

<210> 155
<211> 29
<212> DNA
<213> Homo sapiens

<400> 155
tcacgtccaa catttataga ggcttttgg          29

<210> 156
<211> 27

<212> DNA
<213> Homo sapiens

<400> 156
ttggtcaagc tggtctcaaa ctcctgc          27

<210> 157
<211> 29
<212> DNA
<213> Homo sapiens

<400> 157
acctcaaaat catggcatct ggggtttta          29

<210> 158
<211> 31
<212> DNA
<213> Homo sapiens

<400> 158
taacggaaag cacagaaatg gcaaggttgt g          31

<210> 159
<211> 28
<212> DNA
<213> Homo sapiens

<400> 159
ccatgagggc ctacaagctg gccaccct          28

<210> 160
<211> 25
<212> DNA
<213> Homo sapiens

<400> 160

ggcagggctg gagcttgaca cggtt 25

<210> 161
<211> 26
<212> DNA
<213> Homo sapiens

<400> 161
ctcactgcaa cctccgcctg ctgggt 26

<210> 162
<211> 25
<212> DNA
<213> Homo sapiens

<400> 162
tgactcacca cagcctctgc ctccc 25

<210> 163
<211> 30
<212> DNA
<213> Homo sapiens

<400> 163
taacggaggc aggttggaca aacttgatgt 30

<210> 164
<211> 30
<212> DNA
<213> Homo sapiens

<400> 164
tgctgttaat ccagagtctg tcagcttctt 30

<210> 165
<211> 30

<212> DNA
<213> Homo sapiens

<400> 165
aggggcatat attttggcat gaggtgtgaa                    30

<210> 166
<211> 26
<212> DNA
<213> Homo sapiens

<400> 166
cccaagatct gcccagttag ccttcc                26

<210> 167
<211> 26
<212> DNA
<213> Homo sapiens

<400> 167
cctgagatct tattgggaag cagctg                26

<210> 168
<211> 28
<212> DNA
<213> Homo sapiens

<400> 168
tcttagacat acggtagccc tccgctca              28

<210> 169
<211> 28
<212> DNA
<213> Homo sapiens

<400> 169

tgtgagtttg agaacatggc tggcaatc          28

<210> 170
<211> 25
<212> DNA
<213> Homo sapiens

<400> 170
tcctttccat agcaacaatc ccacg          25

<210> 171
<211> 31
<212> DNA
<213> Homo sapiens

<400> 171
tttacagctt ttaaactctg tcttctgccc c          31

<210> 172
<211> 26
<212> DNA
<213> Homo sapiens

<400> 172
gagatccagc cactgcactc cagact          26

<210> 173
<211> 30
<212> DNA
<213> Homo sapiens

<400> 173
tgggacagtt gctcaaactc cagtattgca          30

<210> 174
<211> 25

<212> DNA
<213> Homo sapiens

<400> 174
ggccaaataa gggaataaaa gctac 25

<210> 175
<211> 28
<212> DNA
<213> Homo sapiens

<400> 175
acgccattgc actccagcct gagtgata 28

<210> 176
<211> 26
<212> DNA
<213> Homo sapiens

<400> 176
tggtggctca cgcctgtaat cccagc 26

<210> 177
<211> 27
<212> DNA
<213> Homo sapiens

<400> 177
ttatcaaaag cacagtcctt tcatgca 27

<210> 178
<211> 27
<212> DNA
<213> Homo sapiens

<400> 178

tgctaaaaat tcaaatacag atagcaa 27

<210> 179
<211> 27
<212> DNA
<213> Homo sapiens

<400> 179
agtgatgctc cccaaaacta ccaggta 27

<210> 180
<211> 25
<212> DNA
<213> Homo sapiens

<400> 180
tccccaagta gttgggacta cggac 25

<210> 181
<211> 26
<212> DNA
<213> Homo sapiens

<400> 181
ccatgtgccc cctgaatctg aagtta 26

<210> 182
<211> 26
<212> DNA
<213> Homo sapiens

<400> 182
ggcatgaacc caggaggtgg agcttg 26

<210> 183
<211> 25

<212> DNA
<213> Homo sapiens

<400> 183
tcctgacctc gtaatctgcc tgtct          25

<210> 184
<211> 28
<212> DNA
<213> Homo sapiens

<400> 184
taatcccctt gggtctcctt tttcttta          28

<210> 185
<211> 30
<212> DNA
<213> Homo sapiens

<400> 185
ttaaaccagt atttccaaac ttcaaggtta          30

<210> 186
<211> 30
<212> DNA
<213> Homo sapiens

<400> 186
cgacagagcg agactctggc taaaattaaa          30

<210> 187
<211> 27
<212> DNA
<213> Homo sapiens

<400> 187

cctccccagc cctccccagg tgacaca 27

<210> 188
<211> 29
<212> DNA
<213> Homo sapiens

<400> 188
gaccatgggg cagtggctgc tggatgtag 29

<210> 189
<211> 27
<212> DNA
<213> Homo sapiens

<400> 189
aggcaggaaa atcactcgaa cccagga 27

<210> 190
<211> 31
<212> DNA
<213> Homo sapiens

<400> 190
tcagcacttt gggaggccca ggcgataaag g 31

<210> 191
<211> 30
<212> DNA
<213> Homo sapiens

<400> 191
agtggtgcaa tcttggctca ctgcaagttc 30

<210> 192
<211> 27

<212> DNA
<213> Homo sapiens

<400> 192
agcggcagca aaagaaggat tgccact          27

<210> 193
<211> 27
<212> DNA
<213> Homo sapiens

<400> 193
agcgactgtc cactgattta tctctct          27

<210> 194
<211> 29
<212> DNA
<213> Homo sapiens

<400> 194
cgtcagcctc ttctactgtt ttgggaagt          29

<210> 195
<211> 31
<212> DNA
<213> Homo sapiens

<400> 195
cagatacttc agatagcaga ccgctaacat a          31

<210> 196
<211> 28
<212> DNA
<213> Homo sapiens

<400> 196

tcagtaagct atcctcagcc cccttgag          28

<210> 197
<211> 28
<212> DNA
<213> Homo sapiens

<400> 197
aatgggcaaa ataaccagct ggcattat          28

<210> 198
<211> 29
<212> DNA
<213> Homo sapiens

<400> 198
tggtctcgaa ctccttacct caagtgatc          29

<210> 199
<211> 26
<212> DNA
<213> Homo sapiens

<400> 199
cgaggctgca gtaagctgag atcacg          26

<210> 200
<211> 27
<212> DNA
<213> Homo sapiens

<400> 200
aagcgattct tctgcctcag cctcccg          27

<210> 201
<211> 25

<212> DNA
<213> Homo sapiens

<400> 201
tctgttgccc agggtggagt gcagt          25

<210> 202
<211> 30
<212> DNA
<213> Homo sapiens

<400> 202
agtgtgatgc ctccagcttt gttctttttg          30

<210> 203
<211> 24
<212> DNA
<213> Homo sapiens

<400> 203
caatgacact gcgattggat aagc          24

<210> 204
<211> 25
<212> DNA
<213> Homo sapiens

<400> 204
ggtggaagtt gccactctgt gggct          25

<210> 205
<211> 32
<212> DNA
<213> Homo sapiens

<400> 205

ttgggaggcc aaggtgcgtg gactgtttga gc 32

<210> 206
<211> 28
<212> DNA
<213> Homo sapiens

<400> 206
gagaaaagca gcatcaactc aactcggt 28

<210> 207
<211> 26
<212> DNA
<213> Homo sapiens

<400> 207
gacctcaagt gatcagccca cctcag 26

<210> 208
<211> 31
<212> DNA
<213> Homo sapiens

<400> 208
tccacaggct gcaatatagt ggtgcaatca c 31

<210> 209
<211> 29
<212> DNA
<213> Homo sapiens

<400> 209
cgggaggctg aggtgggcgg accacgagg 29

<210> 210
<211> 27

<212> DNA
<213> Homo sapiens

<400> 210
agggaggctg aggcaggaga atcatgt          27

<210> 211
<211> 25
<212> DNA
<213> Homo sapiens

<400> 211
gacggtgagc tacacttgaa ttgag          25

<210> 212
<211> 29
<212> DNA
<213> Homo sapiens

<400> 212
gagtctcgcg gtgtcgccca ggctggagt          29

<210> 213
<211> 31
<212> DNA
<213> Homo sapiens

<400> 213
aggaaaggtt cttacattct ttgggcctgt t          31

<210> 214
<211> 30
<212> DNA
<213> Homo sapiens

<400> 214

tgaaggggcc tggttcagct ttttctccag                    30

<210> 215
<211> 34
<212> DNA
<213> Homo sapiens

<400> 215
tgcggtcagg agtttgggac caggctggac aaca                34

<210> 216
<211> 27
<212> DNA
<213> Homo sapiens

<400> 216
ctcggccaat cttttgtatt tgtagta                27

<210> 217
<211> 30
<212> DNA
<213> Homo sapiens

<400> 217
ctagctgccc ttcccaccta gacagtggtg                    30

<210> 218
<211> 29
<212> DNA
<213> Homo sapiens

<400> 218
ccaggctcgg taggcctgtc gcctctacc                29

<210> 219
<211> 24

<212> DNA
<213> Homo sapiens

<400> 219
ccatcgcccg tcccttcgga acgg 24

<210> 220
<211> 26
<212> DNA
<213> Homo sapiens

<400> 220
ccacctgcct ctgcctccca aagtgc 26

<210> 221
<211> 25
<212> DNA
<213> Homo sapiens

<400> 221
tgcgtctccc tccagccaat aggca 25

<210> 222
<211> 25
<212> DNA
<213> Homo sapiens

<400> 222
ctgggaccct caccacgaat ctcat 25

<210> 223
<211> 27
<212> DNA
<213> Homo sapiens

<400> 223

gggaggtaga ctcggaccgg cccgcgg 27

<210> 224
<211> 25
<212> DNA
<213> Homo sapiens

<400> 224
cctgggctta agtgattctc ccacc 25

<210> 225
<211> 24
<212> DNA
<213> Artificial

<220>
<223> an artificially synthesized primer sequence

<400> 225
cgccagggtt ttcccagtca cgac 24

<210> 226
<211> 23
<212> DNA
<213> Artificial

<220>
<223> an artificially synthesized primer sequence

<400> 226
agcggataac aatttcacac agg 23

SEQUENCE LISTING

<110> GENOFUNCTION, INC.

<120> METHOD OF SEARCHING FOR NOVEL TARGET OF DRUG DISCOVERY

<130> EP47354FZ163pau

<140> EP 05 758 233.0
<141> 2005-07-08

<150> PCT/JP2005/012655
<151> 2005-07-08

<150> JP 2004-203672
<151> 2004-07-09

<160> 226

<170> PatentIn Ver. 2.0

<210> 1
<211> 28
<212> DNA
<213> Homo sapiens

<400> 1
ggaggagtcg ggcgccgagg agtccggc 28

<210> 2
<211> 28
<212> DNA
<213> Homo sapiens

<400> 2
gacggacagc ctatttttgc caatatca 28

<210> 3
<211> 25
<212> DNA
<213> Homo sapiens

<400> 3
tgccccggag ggcggagaac tggga 25

<210> 4
<211> 28
<212> DNA
<213> Homo sapiens

<400> 4
tggagcggct gacacaggag cgcattgc 28

<210> 5
<211> 25
<212> DNA
<213> Homo sapiens

<400> 5
ccgaggagtc cggcccggaa gagtc 25

<210> 6
<211> 28
<212> DNA
<213> Homo sapiens

<400> 6
tgggagggct cgcgcgagtt caccgagc 28

<210> 7
<211> 29
<212> DNA
<213> Homo sapiens

<400> 7
tgtatactct gaaagagcga tgcctccag 29

<210> 8
<211> 28
<212> DNA
<213> Homo sapiens

<400> 8
gctccaggtc tttctgcaca tttgggtg 28

<210> 9
<211> 27
<212> DNA
<213> Homo sapiens

<400> 9
gtgcaatgcg ctcctgtgtc ggtcgat 27

<210> 10
<211> 27
<212> DNA
<213> Homo sapiens

<400> 10
cccgactcct ccccgccgtc ctcttca 27

<210> 11
<211> 25
<212> DNA
<213> Homo sapiens

<400> 11
tcaccgagcg cagcacgggc cgcgg 25

<210> 12
<211> 27
<212> DNA
<213> Homo sapiens

<400> 12
ccaggcggca gcgttgggta gggctgc 27

<210> 13
<211> 25
<212> DNA
<213> Homo sapiens

<400> 13
cctgcctcct ccgcgcctac ctcgg 25

<210> 14
<211> 29
<212> DNA
<213> Homo sapiens

<400> 14
gaggagtccg gccccggaag agtccggcc 29

<210> 15
<211> 28
<212> DNA
<213> Homo sapiens

<400> 15
tccgggccgg actcctcggc gcccgact          28

<210> 16
<211> 34
<212> DNA
<213> Homo sapiens

<400> 16
cctgcctcct ccgcgcctac ctcggcctcg tccc          34

<210> 17
<211> 31
<212> DNA
<213> Homo sapiens

<400> 17
tgaactcgcg cgagccctcc cacatctccc c          31

<210> 18
<211> 29
<212> DNA
<213> Homo sapiens

<400> 18
cctggagcgg ttgacacagg agcgcattg          29

<210> 19
<211> 28
<212> DNA
<213> Homo sapiens

<400> 19
gaggagtccg gcccggaaga gtccggcc          28

<210> 20
<211> 35
<212> DNA
<213> Homo sapiens

<400> 20
tgaactcgcg cgagccctcc caggtcatct tctgc          35

<210> 21
<211> 28
<212> DNA
<213> Homo sapiens

<400> 21
tcagggccgt actcttcgac gcctgcct          28

<210> 22
<211> 31
<212> DNA
<213> Homo sapiens

<400> 22
cccaggtcat cttctgcaat cgcagtccgc g          31

<210> 23
<211> 24
<212> DNA
<213> Homo sapiens

<400> 23
ctctgaaaga gcgatgcctc cagg          24

<210> 24
<211> 24

<212> DNA
<213> Homo sapiens

<400> 24
ccaggtcttt ctgcacattt gggt 24

<210> 25
<211> 24
<212> DNA
<213> Homo sapiens

<400> 25
gaagagccaa aggtgacctc ggta 24

<210> 26
<211> 26
<212> DNA
<213> Homo sapiens

<400> 26
caatgttgac ggacagccta tttttg 26

<210> 27
<211> 27
<212> DNA
<213> Homo sapiens

<400> 27
gcccatcgtg tgtccctgcg tctctga 27

<210> 28
<211> 25
<212> DNA
<213> Homo sapiens

<400> 28
cacagctacc gaggtcacct ttggc 25

<210> 29
<211> 33
<212> DNA
<213> Homo sapiens

<400> 29
tgcatctctg aagagccaaa ggtgacctcg gta 33

<210> 30
<211> 25
<212> DNA
<213> Homo sapiens

<400> 30
tcacctttgg ctcttcagag atgca 25

<210> 31
<211> 24
<212> DNA
<213> Homo sapiens

<400> 31
gggacgaggc cgaggtaggc gcgg 24

<210> 32
<211> 24
<212> DNA
<213> Homo sapiens

<400> 32
cctggcacgg gccgccgcat ccac 24

<210> 33
<211> 30
<212> DNA
<213> Homo sapiens

<400> 33
tcttcagggc cgtactcttc gacgcctgcc                    30

<210> 34
<211> 26
<212> DNA
<213> Homo sapiens

<400> 34
tggatatatc taagcccttc tgcaca                26

<210> 35
<211> 24
<212> DNA
<213> Homo sapiens

<400> 35
tgttttcttc ccttatctta ctgt                24

<210> 36
<211> 24
<212> DNA
<213> Homo sapiens

<400> 36
cagttggtca gccatgctgc aagc                24

<210> 37
<211> 25
<212> DNA
<213> Homo sapiens

<400> 37
gcctaaaaat ccattccaag ttgta                25

<210> 38
<211> 30
<212> DNA
<213> Homo sapiens

<400> 38
acaacatgct gacagttcct aaagaaaact                    30

<210> 39
<211> 29
<212> DNA
<213> Homo sapiens

<400> 39
tccctgaatt tctgacaatg atgtgcttt                29

<210> 40
<211> 25
<212> DNA
<213> Homo sapiens

<400> 40
tgaccaattg actgaagagc agatt                25

<210> 41
<211> 27
<212> DNA
<213> Homo sapiens

<400> 41
gagacacagc agcgctgggc ctactgc          27

<210> 42
<211> 26
<212> DNA
<213> Homo sapiens

<400> 42
gaacacacac tccacccatg ccgagg          26

<210> 43
<211> 29
<212> DNA
<213> Homo sapiens

<400> 43
ctccttaatc ccgtccactg ggttatgga          29

<210> 44
<211> 30
<212> DNA
<213> Homo sapiens

<400> 44
cttccagggc tgagataccg ctggtccaca          30

<210> 45
<211> 29
<212> DNA
<213> Homo sapiens

<400> 45
tgtaggttgg tacttccccc attattcct          29

<210> 46
<211> 31
<212> DNA
<213> Homo sapiens

<400> 46
aatcttcaat actctttaaa agcccacttc c          31

<210> 47
<211> 31
<212> DNA
<213> Homo sapiens

<400> 47
tgagctgaag gaaaagattg atcgccgttc t          31

<210> 48
<211> 27
<212> DNA
<213> Homo sapiens

<400> 48
taatcagtgg tggaagaacg gtctcag          27

<210> 49
<211> 30
<212> DNA
<213> Homo sapiens

<400> 49
tgatggccac tagtggtgga cttgcccgaa          30

<210> 50

<211> 32
<212> DNA
<213> Homo sapiens

<400> 50
tgcagcatca ccagacttca agaatttagg gc                32

<210> 51
<211> 28
<212> DNA
<213> Homo sapiens

<400> 51
ggttgcagtt aactgagatt gcgccact          28

<210> 52
<211> 30
<212> DNA
<213> Homo sapiens

<400> 52
gcaggcgcgc ctggccgcga acccggaccc                30

<210> 53
<211> 26
<212> DNA
<213> Homo sapiens

<400> 53
agcaccctgt tgtggctcta taagca          26

<210> 54
<211> 24
<212> DNA
<213> Homo sapiens

<400> 54
ggctgggatt acaggtgtga gcca          24

<210> 55
<211> 26
<212> DNA
<213> Homo sapiens

<400> 55
ggccccagct aactgtaccg ttagca          26

<210> 56
<211> 24
<212> DNA
<213> Homo sapiens

<400> 56
cccaaagggg ccatctcctg tatg          24

<210> 57
<211> 25
<212> DNA
<213> Homo sapiens

<400> 57
cgatgaggtt tttcacctgt gtgga          25

<210> 58
<211> 27
<212> DNA
<213> Homo sapiens

<400> 58

agaagcccct gcgctggctg cctcata 27

<210> 59
<211> 26
<212> DNA
<213> Homo sapiens

<400> 59
ccatgaagaa gcttcatctc aacctc 26

<210> 60
<211> 28
<212> DNA
<213> Homo sapiens

<400> 60
tgagatgagg aatgaaaaca tcagggtg 28

<210> 61
<211> 27
<212> DNA
<213> Homo sapiens

<400> 61
gaatgaaaac ttgccagttt attctgg 27

<210> 62
<211> 25
<212> DNA
<213> Homo sapiens

<400> 62
tctcaaaacc tcatggcagg ctcca 25

<210> 63
<211> 32
<212> DNA
<213> Homo sapiens

<400> 63
cttctttctc tttggtccct cgcccttgta cc 32

<210> 64
<211> 27
<212> DNA
<213> Homo sapiens

<400> 64
tatgggctct acattgtcag ctacttg 27

<210> 65
<211> 27
<212> DNA
<213> Homo sapiens

<400> 65
gacggattct gttgacactg gtgatca 27

<210> 66
<211> 30
<212> DNA
<213> Homo sapiens

<400> 66
tggcttgtta gaaggctgaa agatgatggc 30

<210> 67
<211> 26
<212> DNA

<213> Homo sapiens

<400> 67
tgggaacagt gtctcctgac ttgtca          26

<210> 68
<211> 31
<212> DNA
<213> Homo sapiens

<400> 68
cctcgtactt ctcctaccac tgctgcccag a          31

<210> 69
<211> 29
<212> DNA
<213> Homo sapiens

<400> 69
ttaatagtca cgtttttgcc ccttctatt          29

<210> 70
<211> 27
<212> DNA
<213> Homo sapiens

<400> 70
gttatgcctc actggtctac cttgttc          27

<210> 71
<211> 29
<212> DNA
<213> Homo sapiens

<400> 71
ctattgccta cgtccctgtc gcccaggtc          29

<210> 72
<211> 20
<212> DNA
<213> Homo sapiens

<400> 72
ccacccttct ttgagacacc          20

<210> 73
<211> 25
<212> DNA
<213> Homo sapiens

<400> 73
agtgtttcaa gaaggcatct ttacc          25

<210> 74
<211> 27
<212> DNA
<213> Homo sapiens

<400> 74
ccatgttttc ttgaggtgcg gcttagt          27

<210> 75
<211> 25
<212> DNA
<213> Homo sapiens

<400> 75
actgaggctg aatccgttat taact          25

<210> 76
<211> 26
<212> DNA
<213> Homo sapiens

<400> 76
ttgagaccat cctggccagc atggtg          26

<210> 77
<211> 27
<212> DNA
<213> Homo sapiens

<400> 77
actgagtgaa agagggggct cccctca          27

<210> 78
<211> 29
<212> DNA
<213> Homo sapiens

<400> 78
catatgcctc cgctacttgc ttgaatttt          29

<210> 79
<211> 26
<212> DNA
<213> Homo sapiens

<400> 79
agggattctc ccgcctcagc ctcctg          26

<210> 80
<211> 24
<212> DNA
<213> Homo sapiens

<400> 80
tgctgtgtgg gtgcggccac gtat          24

<210> 81
<211> 29
<212> DNA
<213> Homo sapiens

<400> 81
ggtgaataga ggacaagcga caggcagtg          29

<210> 82
<211> 29
<212> DNA
<213> Homo sapiens

<400> 82
tccagggcca tgtctgactt ctgaatggc          29

<210> 83
<211> 25
<212> DNA
<213> Homo sapiens

<400> 83
cttactcacc accgtttatc cagtg          25

<210> 84
<211> 28
<212> DNA
<213> Homo sapiens

<400> 84
tcccgcgaag cccaatcgcg aggccgcg          28

<210> 85
<211> 26
<212> DNA
<213> Homo sapiens

<400> 85
gctccagggt ggtggagact gttttc          26

<210> 86
<211> 27
<212> DNA
<213> Homo sapiens

<400> 86
agttcgagac cagcctgatc agcatgg          27

<210> 87
<211> 29
<212> DNA
<213> Homo sapiens

<400> 87
ccgctcgata gtttcagaaa tggtgcgtt          29

<210> 88
<211> 30
<212> DNA
<213> Homo sapiens

<400> 88
cccagttcct ctggggctct cagaatcact          30

<210> 89
<211> 26
<212> DNA
<213> Homo sapiens

<400> 89
tgggaggctg aggcaggaaa accgcc          26

<210> 90
<211> 27
<212> DNA
<213> Homo sapiens

<400> 90
ggttggtgtc agtgattttt gtgagac          27

<210> 91
<211> 26
<212> DNA
<213> Homo sapiens

<400> 91
cgccaggcaa agcaggagtc cactga          26

<210> 92
<211> 27
<212> DNA
<213> Homo sapiens

<400> 92
ccgccgcccg tctcctgtcg cgcagct          27

<210> 93
<211> 27

<212> DNA
<213> Homo sapiens

<400> 93
gcattcacca gaaccagcct tgtctat          27

<210> 94
<211> 26
<212> DNA
<213> Homo sapiens

<400> 94
tgatggcggc ggagtgagaa gttaat          26

<210> 95
<211> 31
<212> DNA
<213> Homo sapiens

<400> 95
cagagggaag tccaagccca tggggttctg g          31

<210> 96
<211> 29
<212> DNA
<213> Homo sapiens

<400> 96
acactaacat atacgcccca ttccctgct          29

<210> 97
<211> 27
<212> DNA
<213> Homo sapiens

<400> 97
tgagtagctg ggactacagg tgcgcac          27

<210> 98
<211> 28
<212> DNA
<213> Homo sapiens

<400> 98
tatacagagc ttcctcacct gttcgggc          28

<210> 99
<211> 28
<212> DNA
<213> Homo sapiens

<400> 99
tctgggtact tggtcagtgt ttgtttgc          28

<210> 100
<211> 30
<212> DNA
<213> Homo sapiens

<400> 100
tgattttgtt tttagcttac tgctgccacc          30

<210> 101
<211> 26
<212> DNA
<213> Homo sapiens

<400> 101
tgaatacatg attacccaca gcggca          26

<210> 102
<211> 27
<212> DNA
<213> Homo sapiens

<400> 102
acttctcctg tccctgggac gtgcgtc 27

<210> 103
<211> 25
<212> DNA
<213> Homo sapiens

<400> 103
tgaactcctg acctcgtgat ccacc 25

<210> 104
<211> 26
<212> DNA
<213> Homo sapiens

<400> 104
ctgggtgagt ttcggggatt gccata 26

<210> 105
<211> 26
<212> DNA
<213> Homo sapiens

<400> 105
tagggactgt tagctggagt ttatta 26

<210> 106
<211> 25
<212> DNA
<213> Homo sapiens

<400> 106
ccccggagcc cccaaaccgc agaca 25

<210> 107
<211> 27
<212> DNA
<213> Homo sapiens

<400> 107
ggctggagtg cagtggcacg atctcgg 27

<210> 108
<211> 28
<212> DNA
<213> Homo sapiens

<400> 108
   tcccccctcc tctgaaacag ctgcctct 28

<210> 109
<211> 26
<212> DNA
<213> Homo sapiens

<400> 109
gggccacaaa gcctgctcca cacctt 26

<210> 110
<211> 26
<212> DNA
<213> Homo sapiens

<400> 110
cccaggacca tagccggaag acggag          26

<210> 111
<211> 29
<212> DNA
<213> Homo sapiens

<400> 111
tgtaaaaagc tctaggtgca caataagca          29

<210> 112
<211> 30
<212> DNA
<213> Homo sapiens

<400> 112
tctgggtaaa attactctgt actagtgtag          30

<210> 113
<211> 24
<212> DNA
<213> Homo sapiens

<400> 113
cccagaacga caacgccata tttc          24

<210> 114
<211> 24
<212> DNA
<213> Homo sapiens

<400> 114
ccaagttcca cagctgcaag gcgt          24

<210> 115
<211> 24
<212> DNA
<213> Homo sapiens

<400> 115
cgatgttgaa gcccagcgtt gggg          24

<210> 116
<211> 25
<212> DNA
<213> Homo sapiens

<400> 116
ccctgggcac tggcctagag cctca          25

<210> 117
<211> 28
<212> DNA
<213> Homo sapiens

<400> 117
tggaaagaac catgtggaaa tggaatag          28

<210> 118
<211> 31
<212> DNA
<213> Homo sapiens

<400> 118
taaatgtggc tgatgatgca gttgcttagc t          31

<210> 119

<211> 26
<212> DNA
<213> Homo sapiens

<400> 119
ggcaatgtcg aatctatgag cttcga          26

<210> 120
<211> 30
<212> DNA
<213> Homo sapiens

<400> 120
gaaatagaag cccagagaga ggtaggtgta          30

<210> 121
<211> 27
<212> DNA
<213> Homo sapiens

<400> 121
aactgccaga gttttctctg ggtgtta          27

<210> 122
<211> 29
<212> DNA
<213> Homo sapiens

<400> 122
aacgcctccc ggctctcgtc agcctccca          29

<210> 123
<211> 28
<212> DNA
<213> Homo sapiens

<400> 123
actatctctt ttaactggaa ccaaacca          28

<210> 124
<211> 30
<212> DNA
<213> Homo sapiens

<400> 124
taatttgcct tcccctactc tcttcccatc          30

<210> 125
<211> 26
<212> DNA
<213> Homo sapiens

<400> 125
caccgtgggt tgtttccatt gggaca          26

<210> 126
<211> 26
<212> DNA
<213> Homo sapiens

<400> 126
agaatggcgt cacgtagtcg gtgtgc          26

<210> 127
<211> 26
<212> DNA
<213> Homo sapiens

<400> 127

agcctccttc agcagctact gagcta 26

<210> 128
<211> 27
<212> DNA
<213> Homo sapiens

<400> 128
agagagcaga agccatgaac tgtaagg 27

<210> 129
<211> 26
<212> DNA
<213> Homo sapiens

<400> 129
ggtctcgaac tcctgacttc aagtga 26

<210> 130
<211> 28
<212> DNA
<213> Homo sapiens

<400> 130
ggtgagctgg atattccact cggggcct 28

<210> 131
<211> 27
<212> DNA
<213> Homo sapiens

<400> 131
taggctggtc tcaaactcct gggctca 27

<210> 132
<211> 25
<212> DNA
<213> Homo sapiens

<400> 132
aattaggtaa gattgggaca gaatt 25

<210> 133
<211> 26
<212> DNA
<213> Homo sapiens

<400> 133
tcctttagcg gtcattgcct tctgac 26

<210> 134
<211> 28
<212> DNA
<213> Homo sapiens

<400> 134
ggggatggtc tcgccgcctt ggcaggga 28

<210> 135
<211> 30
<212> DNA
<213> Homo sapiens

<400> 135
gccgagatca cgccactgca cttcagcctg 30

<210> 136
<211> 26
<212> DNA

<213> Homo sapiens

<400> 136
cccaatctta ccagatgcta ctggac          26

<210> 137
<211> 30
<212> DNA
<213> Homo sapiens

<400> 137
cggccgggca acatgctcct ctgcgttggt          30

<210> 138
<211> 31
<212> DNA
<213> Homo sapiens

<400> 138
tggggcgcgg tggctcacgc ctgtggtctc a          31

<210> 139
<211> 33
<212> DNA
<213> Homo sapiens

<400> 139
tatcctgaca atgttcctgt attttttctg gat          33

<210> 140
<211> 26
<212> DNA
<213> Homo sapiens

<400> 140
gccaaggaca ctgatgcctc cccttt          26

<210> 141
<211> 33
<212> DNA
<213> Homo sapiens

<400> 141
cccatgatat aggaggcatt gacgtcgcgg ccg          33

<210> 142
<211> 27
<212> DNA
<213> Homo sapiens

<400> 142
   agttggcctt ggtccttttc tttcggg          27

<210> 143
<211> 27
<212> DNA
<213> Homo sapiens

<400> 143
ccccattgct ctgtgggcgc cccgccc          27

<210> 144
<211> 29
<212> DNA
<213> Homo sapiens

<400> 144
cacccgccat cacgcccggc taaggcttc          29

<210> 145
<211> 26
<212> DNA
<213> Homo sapiens

<400> 145
ccttcgtctc caacctccag gggcag                26

<210> 146
<211> 25
<212> DNA
<213> Homo sapiens

<400> 146
gcctgtgcac cggcagggga gaagg                25

<210> 147
<211> 28
<212> DNA
<213> Homo sapiens

<400> 147
ggttgcagtt ttaaattggg gaatttga                28

<210> 148
<211> 30
<212> DNA
<213> Homo sapiens

<400> 148
gaaatgaatg agcttctgac tttacaggat                30

<210> 149
<211> 25
<212> DNA
<213> Homo sapiens

<400> 149
ggcgagagcc accgcaccca gcccc                25

<210> 150
<211> 30
<212> DNA
<213> Homo sapiens

<400> 150
agggaaaaca gagagagggt caggcctagt                30

<210> 151
<211> 27
<212> DNA
<213> Homo sapiens

<400> 151
tggggtaatc agaggcagcg ctccaga                27

<210> 152
<211> 25
<212> DNA
<213> Homo sapiens

<400> 152
gccttaacct cccaaattgc tgcga                25

<210> 153
<211> 29
<212> DNA
<213> Homo sapiens

<400> 153
cctgaatcac agagtagtac ccttccttg          29

<210> 154
<211> 28
<212> DNA
<213> Homo sapiens

<400> 154
gtagattcac agtgctccga agatcggc          28

<210> 155
<211> 29
<212> DNA
<213> Homo sapiens

<400> 155
tcacgtccaa catttataga ggcttttgg          29

<210> 156
<211> 27
<212> DNA
<213> Homo sapiens

<400> 156
ttggtcaagc tggtctcaaa ctcctgc          27

<210> 157
<211> 29
<212> DNA
<213> Homo sapiens

<400> 157
acctcaaaat catggcatct ggggtttta          29

<210> 158
<211> 31
<212> DNA
<213> Homo sapiens

<400> 158
taacggaaag cacagaaatg gcaaggttgt g          31

<210> 159
<211> 28
<212> DNA
<213> Homo sapiens

<400> 159
ccatgagggc ctacaagctg gccaccct          28

<210> 160
<211> 25
<212> DNA
<213> Homo sapiens

<400> 160
ggcagggctg gagcttgaca cggtt          25

<210> 161
<211> 26
<212> DNA
<213> Homo sapiens

<400> 161
ctcactgcaa cctccgcctg ctgggt          26

<210> 162
<211> 25

<212> DNA
<213> Homo sapiens

<400> 162
tgactcacca cagcctctgc ctccc                25

<210> 163
<211> 30
<212> DNA
<213> Homo sapiens

<400> 163
taacggaggc aggttggaca aacttgatgt                30

<210> 164
<211> 30
<212> DNA
<213> Homo sapiens

<400> 164
tgctgttaat ccagagtctg tcagcttctt                30

<210> 165
<211> 30
<212> DNA
<213> Homo sapiens

<400> 165
aggggcatat attttggcat gaggtgtgaa                30

<210> 166
<211> 26
<212> DNA
<213> Homo sapiens

<400> 166
cccaagatct gcccagttag ccttcc                26

<210> 167
<211> 26
<212> DNA
<213> Homo sapiens

<400> 167
cctgagatct tattgggaag cagctg                26

<210> 168
<211> 28
<212> DNA
<213> Homo sapiens

<400> 168
tcttagacat acggtagccc tccgctca                28

<210> 169
<211> 28
<212> DNA
<213> Homo sapiens

<400> 169
tgtgagtttg agaacatggc tggcaatc                28

<210> 170
<211> 25
<212> DNA
<213> Homo sapiens

<400> 170
tcctttccat agcaacaatc ccacg                25

<210> 171
<211> 31
<212> DNA
<213> Homo sapiens

<400> 171
tttacagctt ttaaactctg tcttctgccc c                31

<210> 172
<211> 26
<212> DNA
<213> Homo sapiens

<400> 172
gagatccagc cactgcactc cagact                26

<210> 173
<211> 30
<212> DNA
<213> Homo sapiens

<400> 173
tgggacagtt gctcaaactc cagtattgca                30

<210> 174
<211> 25
<212> DNA
<213> Homo sapiens

<400> 174
ggccaaataa gggaataaaa gctac                25

<210> 175
<211> 28
<212> DNA
<213> Homo sapiens

<400> 175
acgccattgc actccagcct gagtgata                28

<210> 176
<211> 26
<212> DNA
<213> Homo sapiens

<400> 176
tggtggctca cgcctgtaat cccagc                26

<210> 177
<211> 27
<212> DNA
<213> Homo sapiens

<400> 177
ttatcaaaag cacagtcctt tcatgca                27

<210> 178
<211> 27
<212> DNA
<213> Homo sapiens

<400> 178
tgctaaaaat tcaaatacag atagcaa                27

<210> 179
<211> 27
<212> DNA
<213> Homo sapiens

<400> 179
agtgatgctc cccaaaacta ccaggta                27

<210> 180
<211> 25
<212> DNA
<213> Homo sapiens

<400> 180
tccccaagta gttgggacta cggac                  25

<210> 181
<211> 26
<212> DNA
<213> Homo sapiens

<400> 181
ccatgtgccc cctgaatctg aagtta                 26

<210> 182
<211> 26
<212> DNA
<213> Homo sapiens

<400> 182
ggcatgaacc caggaggtgg agcttg                 26

<210> 183
<211> 25
<212> DNA
<213> Homo sapiens

<400> 183
tcctgacctc gtaatctgcc tgtct                  25

<210> 184
<211> 28
<212> DNA
<213> Homo sapiens

<400> 184
taatcccctt gggtctcctt tttcttta               28

<210> 185
<211> 30
<212> DNA
<213> Homo sapiens

<400> 185
ttaaaccagt atttccaaac ttcaaggtta                          30

<210> 186
<211> 30
<212> DNA
<213> Homo sapiens

<400> 186
cgacagagcg agactctggc taaaattaaa                          30

<210> 187
<211> 27
<212> DNA
<213> Homo sapiens

<400> 187
cctccccagc cctccccagg tgacaca                27

<210> 188

<211> 29
<212> DNA
<213> Homo sapiens

<400> 188
gaccatgggg cagtggctgc tggatgtag 29

<210> 189
<211> 27
<212> DNA
<213> Homo sapiens

<400> 189
aggcaggaaa atcactcgaa cccagga 27

<210> 190
<211> 31
<212> DNA
<213> Homo sapiens

<400> 190
tcagcacttt gggaggccca ggcgataaag g 31

<210> 191
<211> 30
<212> DNA
<213> Homo sapiens

<400> 191
agtggtgcaa tcttggctca ctgcaagttc 30

<210> 192
<211> 27
<212> DNA
<213> Homo sapiens

<400> 192
agcggcagca aaagaaggat tgccact 27

<210> 193
<211> 27
<212> DNA
<213> Homo sapiens

<400> 193
agcgactgtc cactgattta tctctct 27

<210> 194
<211> 29
<212> DNA
<213> Homo sapiens

<400> 194
cgtcagcctc ttctactgtt ttgggaagt 29

<210> 195
<211> 31
<212> DNA
<213> Homo sapiens

<400> 195
cagatacttc agatagcaga ccgctaacat a 31

<210> 196
<211> 28
<212> DNA
<213> Homo sapiens

<400> 196

tcagtaagct atcctcagcc cccttgag 28

<210> 197
<211> 28
<212> DNA
<213> Homo sapiens

<400> 197
aatgggcaaa ataaccagct ggcattat 28

<210> 198
<211> 29
<212> DNA
<213> Homo sapiens

<400> 198
tggtctcgaa ctccttacct caagtgatc 29

<210> 199
<211> 26
<212> DNA
<213> Homo sapiens

<400> 199
cgaggctgca gtaagctgag atcacg 26

<210> 200
<211> 27
<212> DNA
<213> Homo sapiens

<400> 200
aagcgattct tctgcctcag cctcccg 27

<210> 201
<211> 25
<212> DNA
<213> Homo sapiens

<400> 201
tctgttgccc agggtggagt gcagt 25

<210> 202
<211> 30
<212> DNA
<213> Homo sapiens

<400> 202
agtgtgatgc ctccagcttt gttctttttg 30

<210> 203
<211> 24
<212> DNA
<213> Homo sapiens

<400> 203
caatgacact gcgattggat aagc 24

<210> 204
<211> 25
<212> DNA
<213> Homo sapiens

<400> 204
ggtggaagtt gccactctgt gggct 25

<210> 205
<211> 32
<212> DNA

<213> Homo sapiens

<400> 205
ttgggaggcc aaggtgcgtg gactgtttga gc          32

<210> 206
<211> 28
<212> DNA
<213> Homo sapiens

<400> 206
gagaaaagca gcatcaactc aactcggt          28

<210> 207
<211> 26
<212> DNA
<213> Homo sapiens

<400> 207
gacctcaagt gatcagccca cctcag          26

<210> 208
<211> 31
<212> DNA
<213> Homo sapiens

<400> 208
tccacaggct gcaatatagt ggtgcaatca c          31

<210> 209
<211> 29
<212> DNA
<213> Homo sapiens

<400> 209
cgggaggctg aggtgggcgg accacgagg          29

<210> 210
<211> 27
<212> DNA
<213> Homo sapiens

<400> 210
agggaggctg aggcaggaga atcatgt          27

<210> 211
<211> 25
<212> DNA
<213> Homo sapiens

<400> 211
gacggtgagc tacacttgaa ttgag          25

<210> 212
<211> 29
<212> DNA
<213> Homo sapiens

<400> 212
gagtctcgcg gtgtcgccca ggctggagt          29

<210> 213
<211> 31
<212> DNA
<213> Homo sapiens

<400> 213
aggaaaggtt cttacattct ttgggcctgt t          31

<210> 214
<211> 30
<212> DNA
<213> Homo sapiens

<400> 214
tgaaggggcc tggttcagct ttttctccag                    30

<210> 215
<211> 34
<212> DNA
<213> Homo sapiens

<400> 215
tgcggtcagg agtttgggac caggctggac aaca               34

<210> 216
<211> 27
<212> DNA
<213> Homo sapiens

<400> 216
ctcggccaat cttttgtatt tgtagta               27

<210> 217
<211> 30
<212> DNA
<213> Homo sapiens

<400> 217
ctagctgccc ttcccaccta gacagtggtg                    30

<210> 218
<211> 29
<212> DNA
<213> Homo sapiens

<400> 218
ccaggctcgg taggcctgtc gcctctacc               29

<210> 219
<211> 24
<212> DNA
<213> Homo sapiens

<400> 219
ccatcgcccg tcccttcgga acgg               24

<210> 220
<211> 26
<212> DNA
<213> Homo sapiens

<400> 220
ccacctgcct ctgcctccca aagtgc               26

<210> 221
<211> 25
<212> DNA
<213> Homo sapiens

<400> 221
tgcgtctccc tccagccaat aggca               25

<210> 222
<211> 25
<212> DNA
<213> Homo sapiens

<400> 222
ctgggaccct caccacgaat ctcat                25

<210> 223
<211> 27
<212> DNA
<213> Homo sapiens

<400> 223
gggaggtaga ctcggaccgg cccgcgg              27

<210> 224
<211> 25
<212> DNA
<213> Homo sapiens

<400> 224
cctgggctta agtgattctc ccacc                25

<210>  225
<211>  24
<212>  DNA
<213>  Artificial

<220>
<223>  an artificially synthesized primer sequence

<400>  225
cgccagggtt ttcccagtca cgac                 24


<210>  226
<211>  23
<212>  DNA
<213>  Artificial

<220>
<223>  an artificially synthesized primer sequence

<400>  226
agcggataac aatttcacac agg                  23

## Description

### Technical Field

The present invention relates to siRNA expression systems that suppress gene expression using RNAi effects, libraries thereof, and methods for comprehensive functional analysis of genes and for discovering drug target molecules using the libraries. The present invention further relates to genes involved in angiogenesis that are obtained as a result of studies using the libraries.

### Background Art

The sequence information of the entire human genome can now be used. Thus, along with the clarification of gene function through identification of human functional genes by comprehensive screening, the discovery of novel drug target molecules based on an understanding of gene function has also become important. Pharmaceutical development requires great risk and much time. Seven to twelve years and an investment of about thirty billion yen are said to be required for the completion of a single pharmaceutical product, and of the candidate compounds that enter clinical testing, about one twelfth are said to launch on the market (Non-patent Document 1). Instead of this kind of conventional drug discovery method that depends on luck, genomic drug discovery, which actively uses information on various genes, has been drawing attention as a rational drug discovery method. DNA microarrays (Non-patent Document 2) and yeast two-hybrid systems (Non-patent Document 3) are widely used as comprehensive methods of screening for drug target molecules in genomic drug discovery. DNA microarray techniques are based on the changes in gene expression levels that occur during various intracellular processes. However, it is difficult to identify functional genes that undergo qualitative alterations, such as phosphorylation, which result in phenotypic changes without altering the expression level of a particular gene. The yeast two-hybrid system provides a powerful method for detecting direct *in vivo* protein-protein interactions, and for identifying genes whose products interact with particular proteins obtained from the cDNA libraries. However, this method is limited in that the predicted interactions do not necessarily reflect the roles played in the phenomenon, and false-positive results frequently arise. Thus, there has been demand for the development of a system for rapidly identifying the crucial genes involved in changes to particular phenotypes at the actual cellular level.

To date, there have been two types of systems for rapidly identifying the crucial genes involved in changes to particular phenotypes at the actual cellular level. The first method screens gene functions by comprehensively expressing various genes and then identifying those genes specifically expressed in cells that show the necessary altered phenotypes (Non-patent Document 4). The second method screens gene functions by comprehensively suppressing the expression of various genes, and then identifying those genes whose function are specifically inhibited or whose expressions are specifically suppressed in cells that show the necessary altered phenotypes (Non-patent Document 5). In particular, the system of comprehensively suppressing gene expression has been noted as an efficient method for screening drug target molecules, since many drugs suppress the function of drug target genes. Methods using ribozymes were previously used as means for suppressing gene expression (Non-patent Document 6), and recently, RNA interference (abbreviated as "RNAi") has been highlighted as a method that allows more effective suppression of gene expression.

RNAi is a phenomenon that can suppress expression of a target gene, where disruption of a target gene mRNA is induced by introducing cells or such with a double-stranded RNA (hereinafter abbreviated as "dsRNA") that comprises a sense RNA comprising a sequence homologous to the target gene mRNA, and an antisense RNA comprising a sequence complementary thereto. Since RNAi can suppress target gene exression, it has generated attention as a simple gene knockout method, taking the place of conventional and complicated gene destruction methods based on low efficiency homologous recombination. The above-described RNAi was originally discovered in Nematoda (Non-patent Document 7), and to date has been observed not only in Nematoda but also in various organisms, such as plants, Nemathelminthes, fruit flies, Protozoa and the like (Non-patent Documents 8 to 11). Target gene expression has been confirmed as actually suppressed upon introducing exogenous dsRNAs to these organisms. RNAi is also being used as a method for creating knockout individuals.

*In vitro,* dsRNAs are known to target mRNAs upon their cleavage in a lysate of early Drosophila embryos or an extract of cultured Drosophila line S2 cells (Non-patent Documents 12 to 14). *In vitro* RNAi reactions require ATP (Non-patent Document 14).

Recent studies on synthetic RNA duplexes have demonstrated that each strand of an siRNA duplex cleaves a target RNA (Non-patent Document 15). A 3' end having a dinucleotide or trinucleotide protrusion in the siRNA duplex was found to be essential for efficient target cleavage (Non-patent Document 15). Such a 3'-protruding end is characteristic of the products of RNase III cleavage, and in cultured Drosophila S2 line cells a multi-domain RNase III, known as Dicer, is required for the cleavage of dsRNAs into siRNAs (Non-patent Document 16). The siRNAs are then associated with a multi-component nuclease called "RISC", which was identified in Drosophila, and are thought to induce enzymes involved in sequence-specific mRNA degradation (Non-patent Documents 13, 16, and 17).

RNAi provides methods for inactivating target genes, and thus provides powerful tools for studying gene functions in *C. elegans, Drosophila,* and plants. Specific inhibition of gene expression can also be achieved through the stable and inducible expression of dsRNAs in animals or plants (Non-patent Documents 18, 19, and 10). The inactivation of genes by dsRNA has been successfully achieved in mouse embryonic carcinoma (EC) cells and embryonic stem (ES) cells (Non-patent Documents 20 an 21); however, RNAi induction by long dsRNAs in cultured mammalian cells is generally not very successful. These failures can be easily explained by the actions of two latent enzymes that form part of the interferon (IFN) defense pathway, which is activated by long dsRNAs (more than 30 base pairs) (Non-patent Document 22). One of these is 2'-5'-oligoadenylate (2-5A) synthetase, which is activated by dsRNAs and enhances the synthesis of 2-5A, which is required for the activation of the sequence-non-specific RNase called RNase L (Non-patent Document 23). The other is protein kinase PKR; its active form phosphorylates eukaryotic initiation factor 2 (eIF2), which is a translational factor, resulting in general inhibition of protein synthesis and leading to cell death (Non-patent Document 24).

Recently, a 21-nucleotide siRNA duplex was reported to specifically suppress the expression of endogenous genes in some types of mammalian cells (Non-patent Document 25). In this case, the 21-nucleotide siRNA duplex can evade the IFN defense system. This finding suggested that an RNAi system or RNAi-related system exists in mammals. Indeed, a number of mammalian homologues of RNAi-related proteins have been identified, such as rde-1, mut-7, and Dicer (Non-patent Documents 26, 27, and 16).

siRNA expression vectors were developed for more convenient use of such siRNAs. The promoters generally used to express siRNAs are PolIII-type, such as H1, U6, and tRNA. For H1 and U6, an siRNA in which the hairpin-structured portion is 21 nucleotides in length is known to show the most suppression of gene expression; however, for tRNA, an siRNA hairpin-structured portion of about 29 nucleotides in length has been shown to suppress gene expression. However, there are no established methods for designing siRNA sequences having a high gene expression suppressing effect. To date, *in-silico* methods have been most frequently attempted, but are insufficiently reliable.

Recently, a method was reported for preparing siRNA expression vector libraries with the aim of comprehensively suppressing the expression of various genes, wherein the library source materials were fragments produced when arbitrary double-stranded DNAs were digested by DNase in a sequence non-specific manner (Non-patent Documents 28 and 29). Each siRNA expression vector clone in the siRNA expression vector library targets some place in the arbitrary double-stranded DNA sequence that was used as its source material. Thus, the library as a whole expresses siRNAs comprehensively, so as to cover the entire sequence of the arbitrary double-stranded DNA used as its source material. This therefore suggests the possibility of comprehensive screening for genes involved in changes in a certain phenotype, *i.e.,* the possibility that drug target genes with high therapeutic effect can be selected from the enormous quantity of drug target gene candidates.

Most previous attempts to use the suppression of expression of particular genes as a drug's pharmacological effect and as a disease treatment, particularly in gene therapy and such, have targeted causative genes, such as oncogenes and viral genes, which are thought to directly cause diseases (Non-patent Document 30). For a complete cure, therapeutic methods targeting causative genes, and gene therapy in particular, require genes to be introduced into every cell of a diseased tissue, which would be impractical considering present gene transfer technologies. For complete cure, it is necessary to introduce the gene into every cell of diseased tissues.
Non-patent Document 1: Allen K., Nature Biotechnol. 16: 1294, 1998
Non-patent Document 2: Kimuzuka F. et al., TANPAKUSHITSU KAKUSAN KOSO (Protein, Nucleic Acid, and Enzyme) 43: 2004, 1998
Non-patent Document 3: Suzuki H. et al., Genome Res. 10: 1758-1765,2001
Non-patent Document 4: Kitamura, T., SAIBO KOGAKU (Cell Technology) 19: 893-901, 2000
Non-patent Document 5: Leo C. et al., Nature Genetics 27 : 23-29, 2001
Non-patent Document 6: Beger C. et al., Proc Natl Acad Sci USA 98: 130-135, 2001
Non-patent Document 7: Fire, A. et al. Potent and specific genetic interference by double-stranded RNA in Caenorhabditis elegans. Nature 391, 806-811, (1998)
Non-patent Document 8: Fire, A. RNA-triggered gene silencing. Trends Genet. 15, 358-363 (1999)
Non-patent Document 9: Sharp, P. A. RNA interference 2001. Genes Dev. 15, 485-490 (2001)
Non-patent Document 10: Hammond, S. M., Caudy, A. A. & Hannon, G J. Post-transcriptional gene silencing by double-stranded RNA. Nature Rev. Genet. 2, 110-119 (2001)
Non-patent Document 11: Zamore, P. D. RNA interference: listening to the sound of silence. Nat Struct Biol. 8, 746-750 (2001)
Non-patent Document 12: Tuschl T., et al., Genes Dev. 13: 3191-3197, 1999
Non-patent Document 13: Hammond S. M., et al., Nature 404: 293-296, 2000
Non-patent Document 14: Zamore P., et al., Cell 101: 25-33, 2000
Non-patent Document 15: Elbashir S. M. et al., Genes Dev. 15: 188-200, 2001
Non-patent Document 16: Bernstein E. et al., Nature 409: 363-366, 2001
Non-patent Document 17: Hammond S. M. et al., Science 293: 1146-1150, 2001
Non-patent Document 18: Kennerdell and Carthew Nature Biotechnol. 18: 896-898, 2000
Non-patent Document 19: Tavernarakis N. et al., Nature Genetics 24: 180-183, 2000
Non-patent Document 20: Billy E. et al., PNAS 98: 14428-14433, 2001
Non-patent Document 21: Paddison P. J. et al., PNAS 99: 1443-1448,2002
Non-patent Document 22: Stark G. R. et al., Annu. Rev. Biochem. 67: 227-264, 1998
Non-patent Document 23: Silverman R. H. in Ribonucleases: Structures and Functions, eds. D'Alessio, G and Riordan J. F. (Academic, New York) pp.515-551
Non-patent Document 24: Clemens M. J. and Elia A., J. Interferon Cytokine Res. 17: 503-524, 1997
Non-patent Document 25: Elbashir S. M. et al., Nature 411: 494-498, 2001
Non-patent Document 26: Tabara H. et al., Cell 99: 123-132, 1999
Non-patent Document 27: Ketting R. F. et al., Cell 99: 133-141, 1999
Non-patent Document 28: Sen, G. et al., Nature Genetics 36: 183-189, 2004
Non-patent Document 29: Shirane, D. et al., Nature Genetics 36: 190-196, 2004
Non-patent Document 30: Amada RG., et al., Hum Gene Ther. 13: 2255-2270, 1999

### Disclosure of the Invention

An objective of the present invention is to provide libraries of expression vectors for stem-loop RNA molecules that enable comprehensive screening of gene function, and to provide comprehensive methods of screening for drug target molecules using these libraries. Another objective of the present invention is to identify drug target molecules expected to have elevated effects upon suppression of expression by these methods. Specifically, an objective of the present invention is to identify factors that regulate the expression of target genes under normal conditions, wherein altered expression of these target genes is expected to have a therapeutic effect, and further to control the expression levels of these genes by suppressing their expression. When these factors suppress the expression of a target gene, expression of that target gene can be enhanced by suppressing the expression of the factors. Alternatively, when such factors enhance the expression of a target gene, the expression of that target gene can be suppressed by suppressing the expression of the factors. These factors are novel drug targets and further, siRNA molecules that suppress the expression of these factors can be applied to pharmaceuticals having novel drug effects. Furthermore, expression vectors for stem-loop RNA molecules that suppress the expression of these factors are thought to be applicable to gene therapy.

The present inventors conducted dedicated studies to achieve the above-described objectives. As a result, the inventors developed libraries for expression vectors of stem-loop RNA molecules that enable comprehensive screening for gene function, and also developed comprehensive methods of screening for drug target molecules using these libraries. Furthermore, by using these methods, drug target molecules expected to have elevated effects upon suppression of their expression were identified.

Specifically, the present invention includes:
[1] a DNA for functional-gene screening that comprehensively screens for a facto r that regulates the expression of a target gene, and which encodes a stem-loop RNA m olecule that has an RNAi effect in a cell and is structured such that a promoter is opera tively linked to a DNA encoding an RNA corresponding to a region arbitrarily selected f rom a known nucleotide sequence and a sequence complementary to the DNA, which are linked in opposite direction *via* a spacer region;
[2] the DNA of [1], wherein the promoter is a PolIII promoter;
[3] the DNA of [2], wherein the promoter is a tRNA promoter;
[4] the DNA of [3], wherein the tRNA promoter is a tRNA^{VAL} promoter;
[5] the DNA of [1], wherein the length of the region arbitrarily selected from th e known nucleotide sequence is 20 bp to 40 bp;
[6] the DNA of [1], wherein the length of the spacer region is 1 bp to 20 bp;
[7] the DNA of [1], wherein the length of the stem region of the expressed ste m-loop RNA molecule is 20 bp to 40 bp;
[8] the DNA of [1], wherein the target gene is a secretory protein;
[9] the DNA of [1], wherein the factor that regulates the expression of the targe t gene is a protein;
[10] a stem-loop RNA molecule that is a transcript of the DNA of any of [1] t o [9] and which produces an RNAi effect in a cell;
[11] an siRNA molecule generated from a transcript of the DNA of any of [1] t o [9] and which produces an RNAi effect in a cell;
[12] a synthetic siRNA molecule that has an RNAi effect in a cell and whose s equence corresponds to that of an siRNA generated in a cell from the transcript of the DNA in any of [1] to [9];
[13] a vector carrying the DNA of any of [1] to [9];
[14] a cell retaining the vector of claim 13 or the DNA of any of [1] to [9];
[15] the cell of [14] that is a mammalian cell;
[16] a composition comprising the vector of [13] or the DNA of any of [1] to [ 9];
[17] a method for producing a cell for functional-gene screening, which comprise s the steps of introducing the vector of [13] or the DNA of any of [1] to [9] into a cel 1, and selecting a cell introduced with the vector;
[18] a method of functional-gene screening that comprehensively screens for a fa ctor that regulates the expression of a target gene, which comprises the steps of:
   (a) introducing the vector of [13] into a cell;
   (b) selecting a cell introduced with the vector;
   (c) analyzing the phenotype of the selected cell; and
   (d) screening for a functional gene based on the sequence of the stem-loop RNA in the vector sequence of the cell found by analyzing the phenotype to have an altered phenoty pe;
[19] the method of functional-gene screening of [18], which further comprises th e step of introducing a cell with a vector comprising a DNA structured such that a pro moter is operatively linked to a DNA encoding the regulatory region for expression of a target gene and a DNA encoding a marker gene that alters the phenotype of the cell;
[20] the method of functional-gene screening of [18] or [19], which identifies a gene that indirectly enhances or suppresses the expression of a target gene;
[21] a method for confirming the function of the identified functional gene of [2 0], which comprises the step of introducing the vector of [13] or the DNA of any of [1 ] to [9] into a cell or experimental animal;
[22] a method for confirming the function of the identified functional gene of [2 0], which comprises the step of introducing the synthetic siRNA molecule of [12] into a cell or experimental animal;
[23] the method of functional-gene screening of [18] or [19], wherein the target gene is a gene associated with hypoxic stimulation;
[24] the method of functional-gene screening of [18] or [19], wherein the target gene is a gene whose expression is enhanced by HIF;
[25] the method of functional-gene screening of [19], wherein the regulatory regi on for expression of the target gene comprises HRE;
[26] a polynucleotide comprising the nucleotide sequence of any of SEQ ID NO s: 1 to 224;
[27] a polynucleotide comprising a 19 bp or more consecutive nucleotide sequen ce region, wherein the nucleotide sequence is arbitrarily selected from any of SEQ ID N Os: 1 to 224;
[28] a polynucleotide encoding a protein associated with hypoxic stimulation, whi ch comprises the nucleotide sequence region of [27];
[29] a protein associated with hypoxic stimulation, which is encoded by the poly nucleotide of [28];
[30] a vector comprising the polynucleotide of any of [26] to [28];
[31] a host cell retaining the vector of [29] or the polynucleotide of any of [26] to [28];
[32] a method for producing the protein of [29], which comprises the step of cu lturing the host cell of [31] and recovering the produced protein from the host cell or c ulture supernatant thereof;and
[33] an siRNA that cleaves the transcript of the polynucleotide of [27].

### Brief Description of the Drawings

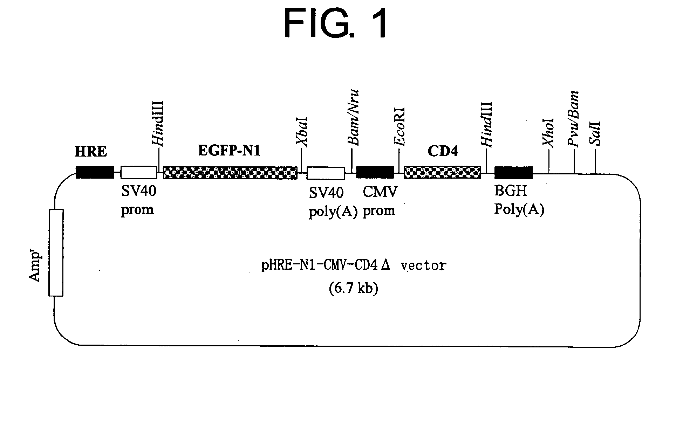

Fig. 1 shows the structure of the pHRE-N1-CMV-CD4Δ vector.

### Best Mode for Carrying Out the Invention

The present invention provides DNAs for screening functional genes, which comprehensively screen for factors that regulate the expression of target genes, and which encode stem-loop RNA molecules with RNAi effect in cells, wherein the DNAs are structured such that a promoter is operatively linked to a DNA encoding an RNA corresponding to a region arbitrarily selected from a known nucleotide sequence, and this is linked to a sequence complementary to that DNA in the opposite direction and *via* a spacer region.

The present invention relates to libraries of expression vectors for siRNA that enable comprehensive screening for gene function, and to comprehensive methods of screening for drug target molecules using these libraries. An siRNA is a double-stranded RNA molecule known to have RNAi effect and commonly of about 20 bp. RNA interference (RNAi) is a phenomenon that can induce the disruption of target gene mRNA and suppress target gene expression when cells or such are introduced with double-stranded RNAs (hereinafter abbreviated as "dsRNAs") that comprise sense RNAs comprising sequences homologous to that of a target gene mRNA, and antisense RNAs comprising complementary sequences thereof.

In the present invention, the libraries of siRNA expression vectors allowing comprehensive screening for gene function are prepared by methods producing at least two or more blunt-ended partial fragments of arbitrary double-stranded DNAs. The methods preferably comprise the steps of:
(a) preparing a double-stranded DNA having at least one or more nicks in each of its two strands by digesting the double-stranded DNA with a nicking enzyme;
(b) digesting the product of step (a) with a single-stranded DNA-specific nuclease and eliminating single-stranded DNAs contained therein; and
(c) preparing a blunt-ended double-stranded DNA by DNA extension using the 3' end of each nick as a synthesis origin and using the 5'-3' polymerase activity of Klenow enzyme.

In the present invention, the libraries of siRNA expression vectors allowing comprehensive screening for gene function comprise two or more fragments prepared as described above, which comprise DNA fragments that each comprise a duplicated DNA region of each other.

In the methods described above, first, DNA fragments are prepared by PCR using double-stranded DNA as a starting material. The double-stranded DNAs for use in the present invention are preferably derived from genomic DNAs, and more preferably from cDNAs. Next, the prepared DNA fragments are digested with a nicking enzyme to prepare small DNA fragments. The nicking enzyme of the present invention is preferably DNase I.

Then, the prepared small DNA fragments are treated with single-stranded DNA-specific nuclease to eliminate the single-stranded DNAs contained therein. Exonuclease VII or Exonuclease I can be used for this treatment.

Next, DNA extension is carried out with the 3' end of each nick as a synthesis origin. For example, Klenow enzyme having 5'-3' polymerase activity may be used for strand replacement synthesis.

The DNAs thus prepared are dephosphorylated by CIP treatment, and blunt-ended double-stranded DNAs are prepared.

The present invention's libraries of siRNA expression vectors that allow comprehensive screening for gene function are prepared by methods for preparing double-stranded DNAs comprising inverted repeats whose repeating unit is a partial DNA fragment from a double-stranded DNA. Specifically, the methods comprise the steps of:
(a) preparing a blunt-ended partial DNA fragment from a double-stranded DNA using the method described above;
(b) dephosphorylating the 5' end of the DNA fragment of step (a);
(c) preparing a DNA molecule structured such that the 5' end of a double-stranded hairpin DNA linker A is ligated to the 5'-dephosphorylated end of the double-stranded DNA of step (b), whereby said linker A binds to both ends of said dephosphorylated double-stranded DNA;
(d) preparing a DNA molecule structured such that the hairpin linker A is linked with the partial DNA fragment of step (a) by extending the DNA using the 3' end of each nick in the DNA molecule of step (c) as synthesis origins, using the 5'-3' polymerase activity of Klenow enzyme;
(e) preparing a DNA molecule structured such that the 3' end of the DNA molecule of step (d) is linked to the 5' end of a double-stranded DNA linker B by ligating the DNA molecule to the 5' end of the linker B; and
(f) preparing a double-stranded DNA with inverted repeat sequences, where the repeating unit is the partial fragment of step (a), by extending the DNA using the 3' end of the nicks as a synthesis origins in the DNA molecule of step (e), using the 5'-3' polymerase activity of Klenow enzyme.

In this method, first, at least two or more of the partial DNA fragments having blunt ends prepared as described above are dephosphorylated at their 5' ends.

Then, the 5' end of the hairpin double-stranded DNA linker A is ligated to prepare DNA molecules structured such that the linker A is linked to each end of a dephosphorylated double-stranded DNA. Examples of linker A include, for example, BL02 linker. The linkers for use in the present methods are preferably phosphorylated at their 5' end. Linker phosphorylation may be achieved enzymatically or by adding a phosphate group to the 5' end at the time of linker synthesis.

T4 DNA ligase can be used for the ligation, for example; however, enzymes with an equivalent function may also be used.

Next, DNA extension is performed by strand replacement synthesis using the 3' end of the nicks in the prepared DNA as synthesis origins, thus preparing DNA molecules structured such that the above-described blunt-ended partial DNA fragments and hairpin-shaped linker A are linked together.

Then, the DNA molecule is ligated to the 5' end of the double-stranded DNA linker B, thus preparing a DNA molecule structured such that the 3' end of the DNA molecule is linked with the 5' end of linker B.

Linker B is a DNA with restriction sites in the DNA region and is preferably a hairpin double-stranded DNA, but does not necessarily have to have a hairpin structure. In the present methods SL02 linker can be used as a hairpin type linker, for example.

Double-stranded DNAs that comprise inverted repeat sequences whose repeating unit is a blunt-ended partial DNA fragment as described above are prepared by DNA extension, carried out by strand-replacement synthesis using the 3' end of the nicks in the DNA molecules prepared as described above as synthesis origins. Enzymes such as Klenow and Bst DNA polymerase can be used for strand-replacement synthesis. Klenow enzyme is used in low-temperature reactions to prevent the dissociation of nucleotide N portions (25 to 35 bp) between two nicks in the DNAs. Bst DNA polymerase can extend faster than Klenow enzyme, and can thus complete the reaction in a shorter time.

The end portion derived from the hairpin linker (SL02) is then cleaved off to prepare the hairpin fragment. *Sac*I is preferably used for cleaving.

The present invention's libraries of siRNA expression vectors that allow comprehensive screening for gene function are prepared by methods of preparing expression vectors for stem-loop RNA molecules, wherein one strand constituting the stem is a transcript of which corresponds to a partial fragment of a double-stranded DNA. Specifically, the methods comprise steps (a) and (b) as follows:
(a) using the procedure described above to prepare a double-stranded DNA comprising inverted repeats whose repeating unit is a partial fragment of a double-stranded DNA; and
(b) preparing an expression vector structured such that the inverted repeat sequence of the DNA of step (a) is placed downstream of a promoter to enable transcription.

A "stem-loop" is also called a "hairpin loop" and refers to a structure in a single-stranded RNA that consists of a double-stranded portion (the stem) formed by hydrogen bonding between inverted repeat sequences, with a loop in between.

In the present methods, first, a DNA comprising inverted repeat sequences whose repeating unit is a partial fragment of a double-stranded DNA, prepared by the procedure described above, is ligated downstream of an expression vector promoter so that the sequence can be transcribed. If the vector in which the sequence was inserted comprises a "stuffer" portion (*Bam*HI*-Bam*HI) within the hairpin loop, the siRNA expression vector library is constructed after this portion is removed. The above-described "stuffer" portion is not limited to the *Bam*HI-*Bam*HI fragment described in the Examples, and is determined depending on the type of restriction enzyme used.

The type, copy number and position of the above promoters may be arbitrarily determined, so long as the promoters enable transcription (expression) of a DNA construct prepared by the above methods of the present invention.

The above promoters include, for example, polII and polIII promoters. In the present invention, preferably used promoters are PolIII promoters, which are suited to the expression of short RNAs, such as siRNAs. Such polIII promoters include, for example, U6 promoter, tRNA promoter, retroviral LTR promoter, adenovirus VAI promoter, 5S rRNA promoter, 7SK RNA promoter, 7SL RNA promoter, H1 RNA promoter and the like. The U6 promoter described above adds four uridine nucleotides to the 3' end of RNAs; the number of nucleotides protruding at the 3' end of the final siRNA product can be deliberately adjusted to 4, 3, 2, 1, or 0 by providing "A" as the first 0, 1, 2, 3, or 4 nucleotides in the antisense- and sense-encoding DNA. Even when other promoters are used, the number of protruding nucleotides at the ends can be deliberately changed in a similar manner.

The polII promoters can include cytomegalovirus promoter, T7 promoter, T3 promoter, SP6 promoter, RSV promoter, EF-1α promoter, β-actin promoter, γ-globulin promoter, and SRα promoter. The RNAs synthesized when using polII systems are moderately long, and not short like the polIII systems. Therefore, when using polII systems, antisense or sense RNAs can be generated from the moderately long synthesized RNAs by using, for example, means able to cleave RNAs by self-processing, such as ribozymes. Stem-loop RNAs can also be generated by inserting a stem-loop sequence immediately after the polII promoter and then inserting a polyA signal behind it.

A tRNA promoter can be preferably used as a promoter of the present invention. Whereas a tRNA promoter is located upstream of the gene in procaryotes, in eukaryotes the DNA region corresponding to a D or T loop of a tRNA is known to function as a tRNA promoter. Thus, a tRNA promoter of the present invention generally refers to the DNA region corresponding to a D or T loop of a tRNA. In general, there are many types of tRNA for each amino acid. A tRNA promoter corresponding to valine (Val) (tRNA^{Val}) can be preferably used as a tRNA promoter of the present invention.

In the present invention, the phrase "operatively linked" means that the DNA and a tRNA promoter are linked together so that the above hairpin transcript is generated through transcription by the tRNA promoter. Accordingly, tRNA promoters may be downstream or upstream of the DNA; however, they are generally located upstream. Meanwhile, any DNA sequence may be placed in between the DNA and a tRNA promoter, as long as the DNA can be transcribed.

In the present invention, as a result of the above transcription by the tRNA promoter, the tRNA itself is linked with the above stem-loop RNA molecule and serves as a cytoplasmic localization signal.

In the present invention, the above DNA may optionally be provided with a terminator. The terminator is not particularly limited, as long as the terminator can stop transcription from the promoter. For example, known terminators can be used, such as sequences of four or more consecutive A (adenine) nucleotides and sequences capable of forming a palindrome structure. The DNAs of the present invention comprises single- and double-stranded DNAs.

In the present invention, the above "region arbitrarily selected from known nucleotide sequences" is preferably 20 to 40 bp long.

Transcription of an above DNA of the present invention by a tRNA promoter generates a sequence of transcripts (RNA molecules). Since the DNAs of the present invention have inverted repeats with a spacer region in between, the transcripts of the DNAs of the present invention also have a structure comprising the inverted repeat sequences with a spacer region in between. RNA molecules with such structures generally form hydrogen bonds between the repeat sequences, forming a stem and loop from the repeat sequences and spacer region respectively. In the present invention, RNA molecules that form such a stem-loop structure are called "stem-loop RNA molecules". Stem-loop RNA molecules transcribed from the above DNAs of the present invention are also included in the present invention.

Furthermore, the siRNA molecules that have an RNAi effect in cells and that are generated in cells from the transcripts of the above DNAs of the present invention, are also included in the present invention. In addition, synthetic siRNA molecules that have sequences corresponding to siRNA molecules that have an RNAi effect in cells and that are generated in the cells from the transcripts of the above DNAs of the present invention and are also included in the present invention. Those skilled in the art can prepare the above "synthetic siRNA molecules" of the present invention accordingly, based on the methods disclosed herein. In this case, when one of the strands is known, those skilled in the art can readily learn the nucleotide sequence of the other strand (the complementary strand). The siRNAs of the present invention can be prepared accordingly by those skilled in the art using commercially available nucleic acid synthesizers. Alternatively, general custom synthesis services may be used to synthesize desired RNAs.

In the present invention, "having RNAi effect" also includes cases where metabolites or such of the stem-loop RNA molecules of the present invention have RNAi effect (for example, products generated *via* metabolism, such as RNA chain cleavage).

The length of a DNA constituting a spacer region of the present invention is not particularly limited, as long as the flanking repeat sequences can form hydrogen bonds; the length is typically one to 20 bases, preferably one to ten bases, more preferably three to eight bases, and still more preferably four to six bases. The nucleotide sequence of a DNA constituting the spacer region is not particularly limited and can be an arbitrary sequence. A spacer region is not necessary required, as long as hydrogen bonds can be formed between inverted repeat sequences, as described above; DNAs with no spacer region are also included in the DNAs of the present invention.

Usually, when a double-stranded RNA of about 40 bp or longer is introduced into mammalian cells, the double-stranded RNA is known to be digested by the interferon (IFN) defense pathway. Thus, the length of the double-stranded RNA stem region of a stem-loop RNA molecule of the present invention is typically within 40 bp, and preferably 20bp to 40 bp.

Those skilled in the art can prepare the above DNAs of the present invention using common genetic engineering techniques. The DNAs of the present invention comprising tRNA promoters can be prepared, for example, by synthesizing arbitrary sequences using known oligonucleotide synthesis methods.

The DNAs of the present invention may be expressed in cells by introducing the DNAs as it is into the cell chromosomes; however, the above DNAs are preferably carried by vectors for efficient gene transfer into cells, and the like. Vectors comprising the DNAs of the present invention are also included in the present invention. The "vectors" that can be used herein can be selected depending on the cells and such to be introduced with that vector. For example, vectors for mammalian cells include viral vectors, for example, retroviral vectors, adenoviral vectors, adeno-associated virus vectors, vaccinia virus vectors, lentivirus vectors, herpes virus vectors, alpha-virus vectors, EB virus vectors, papilloma virus vectors, foamy virus vectors and such; and non-viral vectors, such as cationic liposomes, ligand-DNA complexes, and gene guns (Niitsu Y. et al., Molecular Medicine 35: 1385-1395 (1998)), but are not limited thereto. Furthermore, instead of viral vectors, it is also preferable to use dumbbell DNAs (Zanta M.A. et al., Gene delivery: a single nuclear localization signal peptide is sufficient to carry DNA to the cell nucleus. Proc Natl Acad Sci U.S.A. 1999 Jan 5;96(1):91-6), modified DNAs with nuclease resistance, and naked plasmids (Liu F, Huang L. Improving plasmid DNA-mediated liver gene transfer by prolonging its retention in the hepatic vasculature. J. Gene Med. 2001 Nov-Dec;3(6):569-76).

The vectors may further have a selection marker or the like, as required, which allows selection of cells that have been introduced with the vectors. Such selection markers include drug resistance markers, such as neomycin resistance gene, hygromycin resistance gene, and puromycin resistance gene; markers that allow selection using enzymatic activity as an indicator, such as galactosidase; or markers that enable selection using fluorescence, luminescence, and the like as an indicator, such as GFP. Alternatively, selection markers that allow selection using surface antigens as indicators, such as EGF receptor and B7-2, can also be used. The use of such selection markers thus allows selection of only those cells introduced with the vectors, namely, those cells introduced with a vector of the present invention. In addition, using vectors prolongs the intracellular retention time, and since vectors such as retroviral vectors induce the integration into chromosomes, it becomes possible to stably supply stem-loop RNA molecules from the DNAs of the present invention in cells, depending on the vector.

In the present invention, the above "target genes" are, for example, secretory proteins, while the above "factors that regulates target gene expression" are, for example, proteins.

In a preferred embodiment of the present invention, gene expression in a cell can be suppressed by introducing a DNA of the present invention or a vector comprising the DNA into the cell. The present invention thus provides methods for producing cells in which target gene expression is suppressed, where the methods comprise the steps of introducing cells with a DNA of the present invention or a vector comprising the DNA and selecting the cells introduced with the above-mentioned vector. The present invention also provides cells retaining a DNA of the present invention or a vector comprising that DNA. The above cells of the present invention are not particularly limited and desired cells in which suppression of gene expression is intended can be used. The DNAs of the present invention or vectors comprising these DNAs enables RNAi induction, even in mammalian cells for which RNAi induction had previously been difficult; thus, mammalian cells can be preferably used as the cells of the present invention. Cells such as plant cells in which retention of long-term stable expression is difficult with long dsRNAs are also preferable as the cells to be introduced with the DNAs of the present invention or vectors comprising these DNAs.

Those skilled in the art can select appropriate methods for introducing the above cells with a DNA of the present invention or a vector comprising that DNA, depending on the type of cells. For example, for gene transfer to mammalian cells selection can be from calcium phosphate methods (Virology, Vol.52, p.456 (1973)), electroporation (Nucleic Acids Res., Vol.15, p.1311 (1987)), lipofection (J. Clin. Biochem. Nutr., Vol.7, p.175 (1989)), gene transfer methods based on viral infection (Sci.Am., p.34, March (1994)), gene guns, and the like. Introduction into plant cells can be performed by electroporation (Nature, Vol.319, p.791 (1986)), polyethylene glycol methods (EMBO J., Vol.3, p.2717 (1984)), particle gun ( Proc. Natl. Acad. Sci. USA, Vo 1.85, p.8502 (1988)), *Agrobacterium*-mediated methods (Nucleic. Acids Res., Vol.12, p.8711 (1984)), or such.

Cells introduced with a DNA of the present invention or a vector comprising that DNA can be selected using known methods, such as hybridization and PCR, using as a probe or primer a DNA sequence specific to a DNA of the present invention or a vector comprising that DNA. Alternatively, when a DNA of the present invention is carried by a vector with a selection marker, the cells can be selected using phenotypes due to the selection marker as an indicator.

The above cells introduced with a DNA of the present invention, or a vector comprising that DNA, are knockdown cells, in which target gene expression is suppressed. Herein, "knockdown cells" include cells in which target gene expression is completely suppressed and cells in which target gene expression is not completely suppressed but is reduced. Previously, such cells have been created by deleting or altering target genes or regulatory regions thereof; however, by using the present invention, cells in which target gene expression is suppressed can be prepared by a simple method that comprises simply introducing a DNA of the present invention or a vector comprising that DNA and selecting the introduced cells, without changing target genes on the chromosome. The knockdown cells prepared in this way can be used as materials for research into the functional analyses of target genes, and cells where the expression of a target gene that is a causative gene of a disease is suppressed can be used as cellular models for the disease, or such. Furthermore, target gene-knockdown animals, model animals for diseases, and such can be created by introducing germ cells with a DNA of the present invention or a vector comprising that DNA and then developing a biological organism from the germs cells comprising this present system. The present invention also includes the above knockdown cells produced according to the present invention.

The present invention also relates to compositions comprising a DNA of the present invention or a vector comprising that DNA. The present invention can suppress the expression of desired target genes, and therefore therapeutic or prophylactic effects on a disease are anticipated upon using the present invention to suppress the expression of a gene causing that disease. In addition, the present invention identifies factors that regulate expression of target genes under normal conditions, where altering the expression level of a target gene is expected to have a therapeutic effect, and it further enables control of the level of gene expression by suppressing the expression of the factors. When such a factor suppresses target gene expression, suppressing the expression of that factor can enhance the expression of the target gene. Alternatively, when the factor enhances target gene expression, suppressing the expression of that factor can suppress the expression of the target gene. Such factors are novel drug targets; at the same time, siRNA molecules that suppress the expression of these factors can be applied to pharmaceuticals having novel drug effects. In addition, expression vectors for stem-loop RNA molecules that suppress the expression of these factors would be applicable to gene therapy.

Furthermore, the above compositions are also useful as reagents to examine functions of desired genes. When used as pharmaceuticals, the DNAs of the present invention or vectors comprising these DNA may be formulated as compositions by adding suitable excipients and such.

In another embodiment, the present invention relates to expression vectors for stem-loop RNA molecules. These vectors comprise DNAs structured such that a DNA encoding an RNA consisting of an arbitrary sequence is linked in the opposite direction to a sequence complementary to that DNA *via* a spacer region, which is then operatively linked to a tRNA promoter. Herein, transcripts expressed from such DNAs are called "stem-loop RNA molecules". Functional genes can be screened by introducing the stem-loop RNA molecules into cells. Specifically, thus far DNAs of the present invention or vectors comprising these DNAs have been able to suppress the expression of particular target genes; however, in the above embodiment, the DNAs can be used to screen novel functional genes by expressing the stem-loop RNAs and suppressing arbitrary genes, for example, genes with unknown functions or sequences.

The present invention provides methods of screening for functional genes and drug target molecules, which comprise the steps of introducing the above expression vectors for stem-loop RNA molecules into cells, selecting cells introduced with the vectors, and analyzing phenotypes of the selected cells.

The methods described above preferably comprise the step of analyzing functional genes based on stem-loop RNA sequences within the sequences of vectors in cells whose phenotypes have been analyzed and were found to be altered.

In the present invention, libraries can also be constructed by collecting expression vectors that can express stem-loop RNA molecules with different sequences. Functional genes can be screened more efficiently by using these libraries.

In the above methods, the expression vectors for stem-loop RNA molecules can be introduced into the cells as described above.

In the above methods, cell phenotyping is carried out after selecting cells that were introduced with the expression vectors for stem-loop RNA molecules or libraries. Phenotypes can be analyzed by, for example, comparison with the phenotype of control cells not introduced with the expression vectors for stem-loop RNA molecules. These phenotypes include not only changes which occur on the cell-surface, but also, for example, intracellular alterations.

Cells found to have altered phenotypes as a result of the above analysis very probably comprise stem-loop RNA molecules that can suppress the expression of certain functional genes. Thus, to screen for functional genes, for example, probes or primers are prepared based on the DNA sequences encoding the stem-loop RNAs in the expression vectors for stem-loop RNA molecules that are in these cells. Then, the functional genes can be cloned by hybridization or PCR using these probes or primers. Alternatively, databases can be searched for the functional genes based on the DNA sequences encoding the stem-loop RNAs.

The present invention also provides methods for confirming the functions of genes identified by the above methods of screening for functional genes. Specifically, the methods comprise the step of introducing an above DNA or vector of the present invention into cells or experimental animals or introducing a synthetic siRNA molecule of the present invention into cells or experimental animals. The experimental animals can include, for example, dogs, rats, hamsters, rabbits, pigs, cows, horses, monkeys, sheep, goats, cats and the like.

The present inventors also provided partial polynucleotide fragments of polynucleotides encoding proteins involved in angiogenesis based on hypoxic stimulation. The above polynucleotide sequences of the present invention are shown in SEQ ID NOs: 1 to 224. Sequences complementary to the sequences of SEQ ID NOs: 1 to 224 are also included in the polynucleotides of the present invention.

The polynucleotides provided by the present inventors are partial sequences of genes involved in angiogenesis based on hypoxic stimulation; however, those skilled in the art can readily isolate full-length cDNAs for genes involved in angiogenesis based on hypoxic stimulation based on the sequence information of any one of the polynucleotides of SEQ ID NOs: 1 to 224. Specifically, a full-length cDNA can be obtained, for example, by hybridization-based methods for screening cDNA libraries or the like prepared from various cells using a sequence of any of SEQ ID NOs: 1 to 224 as a probe; or by methods for screening libraries using a sequence of any of SEQ ID NOs: 1 to 224 as a primer and DNA from various cDNA libraries as a template, with the indicator being the amplification of products with a size specific to the primer. Alternatively, the full-length cDNAs for genes involved in angiogenesis based on hypoxic stimulation can be obtained by RACE, where PCR is carried out using a sequence of any of SEQ ID NOs: 1 to 224 as a primer and where mRNAs prepared from various cells are converted to single-stranded cDNAs and oligomers are attached to its end (Frohman, M. A. et al.: Proc. Natl. Acad. Sci. USA, 85: 8992, 1988). When the polynucleotides of the present invention are used as hybridization probes, labeled polynucleotides are generally used. Labeling methods include, for example, nick translation using DNA polymerase I, terminal labeling using polynucleotide kinase, fill-in terminal labeling using Klenow fragments (Berger SL, Kimmel AR. (1987) Guide to Molecular Cloning Techniques, Method in Enzymology, Academic Press; Hames BD, Higgins SJ (1985) Genes Probes: A Practical Approach. IRL Press; Sambrook J, Fritsch EF, Maniatis T. (1989) Molecular Cloning: a Laboratory Manual, 2nd Edn. Cold Spring Harbor Laboratory Press), labeling through transcription using RNA polymerase (Melton DA, Krieg, PA, Rebagkiati MR, Maniatis T, Zinn K, Green MR. (1984) Nucleic Acid Res., 12,7035-7056), and non-radioisotopic methods of incorporation of modified nucleotides into DNAs (Kricka LJ. (1992) Nonisotopic DNA Probing Techniques. Academic Press).

The present invention provides protein-encoding polynucleotides which comprise a nucleotide sequences of any of SEQ ID NOs: 1 to 224 and are involved in angiogenesis based on hypoxic stimulation. The polynucleotides include full-length cDNAs for genes involved in angiogenesis based on hypoxic stimulation, which can be isolated based on the sequence information of any of SEQ ID NOs: 1 to 224.

The polynucleotides of the present invention also comprise polynucleotides comprising a region of 19 bp or more consecutive nucleotides arbitrarily selected from the sequence of any of SEQ ID NOs: 1 to 224. The present invention also includes siRNAs that cleave the transcripts of polynucleotides of the present invention.

The present invention also includes polynucleotides that specifically hybridize under stringent conditions to polynucleotides comprising a nucleotide sequence of any of SEQ ID NOs: 1 to 224. Typical stringent hybridization conditions are "1x SSC, 0.1% SDS, 37°C" or similar conditions; more stringent conditions are "0.5x SSC, 0.1% SDS, 42°C" or similar; and still more stringent conditions are "0.2x SSC, 0.1% SDS, 65°C" or similar. Thus, the more stringent the hybridization conditions, the more hope of successfully isolating DNAs with high homology to the probe sequence. The above combinations of SSC, SDS, and temperature are examples and those skilled in the art can achieve stringencies similar to the above by appropriately combining the above and other factors that contribute to determining hybridization stringency (for example, probe concentration, probe length, reaction time for hybridization, and the like).

The polynucleotides of the present invention can be used as antisense polynucleotides (antisense DNAs/RNAs; for example, antisense RNAs complementary to the transcripts of genes encoding proteins of the present invention and DNAs encoding the RNAs) for suppressing the expression of the above proteins involved in angiogenesis based on hypoxic stimulation or as polynucleotides constituting ribozymes (or DNAs encoding RNAs with ribozyme activity).

As shown below, the action of the antisense polynucleotides in suppressing target gene expression involves many factors, including: transcription inhibition by triplex formation, transcription suppression by the formation of hybrid with a portion of locally opened loop structure generated by RNA polymerase, transcription inhibition by the formation of a hybrid with an RNA being synthesized, suppression of splicing by the formation of a hybrid at an intron-exon junction, suppression of splicing by the formation of a hybrid with the portion responsible for spliceosome formation, suppression of cytoplasmic translocation from the nucleus by the formation of a hybrid with an mRNA, suppression of splicing by the formation of a hybrid with a capping site or poly(A) site, suppression of translation initiation by the formation of a hybrid with a binding site for a translation initiation factor, suppression of translation by the formation of a hybrid with a ribosome-binding site adjacent to the start codon, inhibition of extension of peptide chain by the formation of a hybrid with a translational region or ribosome-binding site of mRNA, suppression of gene expression by the formation of a hybrid with a nucleic acid-protein interaction site, and the like. These inhibit the process of transcription, splicing, or translation and suppress target gene expression (Hirashima and Inoue, Shin Seikagaku Jikken Koza (New Courses in Experimental Biochemistry) 2, Kakusan (Nucleic Acid) IV: "Idenshi no Fukusei to Hatugen (Replication and expression of genes)", Ed. The Japanese Biochemical Society, Tokyo Kagakudojin, pp. 319-347, 1993).

The antisense polynucleotides for use in the present invention may suppress target gene expression by any of the actions described above. In one embodiment, antisense sequences designed to be complementary to the untranslated region adjacent to the 5' end of an mRNA of a gene would effectively inhibit translation of the gene. Alternatively, sequences complementary to the coding region or 3' untranslated region can also be used. Thus, the antisense polynucleotides used in the present invention include not only polynucleotides comprising antisense sequences of translational regions of genes, but also polynucleotides comprising antisense sequences of untranslated regions. The antisense polynucleotides to be used are linked downstream of an appropriate promoter, and preferably a transcription termination signal is linked to their 3' end. The antisense polynucleotide sequences are preferably sequences complementary to a target gene or portion thereof; however, the sequences do not need to be perfectly complementary as long as they can effectively inhibit gene expression. The transcribed RNA is preferably 90% or more, and more preferably 95% or more complementary to the target gene transcript.

The antisense polynucleotides can be prepared, for example, based on sequence information of the polynucleotides of the present invention (for example, the nucleotide sequence of a gene encoding a protein isolated based on SEQ ID NO: 1), using the phosphorothioate method (Stein, 1988 Physicochemical properties of phosphorothioate oligodeoxynucleotides. Nucleic Acids Res 16, 3209-21 (1988)) or such.

The expression of an endogenous gene can also be suppressed by using polynucleotides encoding ribozymes. There are various ribozymes with different activities; studies of ribozymes that serve as RNA-cleaving enzymes have enabled the design of ribozymes for site-specific RNA cleavage. Ribozymes include those large ribozymes that comprise 400 nucleotides or more, such as group-I intron-type ribozymes and the RNAseP ribozyme M1RNA, as well as ribozymes with an active domain of about 40 nucleotides, called hammerhead- or hairpin-type ribozymes (Makoto Koizumi and Eiko Otsuka, (1990) Tanpakushitu, Kakusan, Koso (Protein, Nucleic acid and Enzyme), 35:2191).

For example, the autocleavage domain of hammerhead-type ribozymes cleaves G13U14C15 at the 3' end of C15. The base pairing of U14 with A at position 9 is important for this activity, and cleavage has been shown to occur even when the nucleotide at position 15 is A or U instead of C (M. Koizumi et al., (1988) FEBS Lett. 228:225). When the substrate-binding site of a ribozyme is designed to be complementary to an RNA sequence adjacent to the target site, a restriction enzyme-like RNA-cleaving ribozyme that recognizes the sequence UC, UU, or UA in the target RNA can be created (M. Koizumi et al., (1988) FEBS Lett. 239:285; Makoto Koizumi and Eiko Otsuka, (1990) Tanpakushitu, Kakusan, Koso (Protein, Nucleic acid and Enzyme), 35:2191; M. Koizumi et al., (1989) Nucleic Acids Res. 17:7059). In the present invention, the transcripts (mRNAs) of the sequences of SEQ ID NOs: 1 to 30 serve as ribozyme target sequences. Therefore, those skilled in the art can readily prepare ribozymes that recognize these sequences as a target sequence. Ribozymes prepared in this way would cleave the transcripts of the present invention's polynucleotides encoding proteins involved in angiogenesis based on hypoxic stimulation. These ribozymes are also included in the present invention.

The present invention also provides proteins encoded by the polynucleotides of the present invention. These proteins are proteins involved in angiogenesis based on hypoxic stimulation. The proteins of the present invention can be produced by any suitable method. Such proteins include naturally occurring proteins that have been isolated, proteins produced by recombination techniques, proteins produced synthetically, and proteins produced by combination of these methods. Such means for producing proteins are well known to those skilled in the art. The recombinant proteins can be prepared, for example, by introducing appropriate host cells with a vector introduced with a polynucleotide of the present invention and then purifying the protein expressed in the transformant. Naturally occurring proteins can be prepared, for example, using affinity column to which antibodies against the proteins are bound (Current Protocols in Molecular Biology, eds. Ausubel et al. (1987) Publish. John Wiley & Sons, Section 16.1-16.19). Those skilled in the art can prepare antibodies for any proteins by known methods. Antibodies used in affinity purification may be polyclonal or monoclonal antibodies. The proteins of the present invention can also be prepared by *in-vitro* translation or such.

The present invention also provides vectors containing the polynucleotides of the present invention, host cells retaining the polynucleotides or vectors of the present invention, and methods for producing the polypeptides of the present invention by using these host cells.

The present invention also provides methods for producing the proteins of the present invention that comprise the step of culturing the host cells and recovering the produced proteins from the host cells or culture supernatants thereof.

The vectors of the present invention are not particularly limited as long as they stably retain an inserted DNA; for example, when *Escherichia coli* (*E. coli*) is used as the host, preferred cloning vectors include pBluescript vector (Stratagene). When using vectors to produce the polypeptides of the present invention, expression vectors are particularly useful. The expression vectors are not particularly limited as long as the vectors express the polypeptides *in vitro,* in *E. coli,* in culture cells, or in a body of an organism. For example, pBEST vector (Promega) is preferred for *in-vitro* expression; pET vector (Invitrogen) is preferred for *E. coli*; pME18S-FL3 vector (GenBank Accession No. AB009864) is preferred for culture cells; and pME18S vector (Mol Cell Biol. 8:466-472(1988)) is preferred for bodies of organisms. DNAs of the present invention can be inserted into the vectors by conventional methods, for example, by ligation using restriction sites (Current protocols in Molecular Biology, eds. Ausubel et al. (1987) Publish. John Wiley & Sons, Section 11.4-11.11).

The host cells to be introduced with the vectors of the present invention are not particularly limited and various host cells can be used depending on the purpose. Cells to be used for expressing the polypeptides include, for example, bacterial cells (for example, *Streptococcus, Staphylococcus, E. coli, Streptomyces,* and *Bacillus subtilis)*; fungal cells (for example, yeast and *Aspergillus)*; insect cells (for example, Drosophila S2 and Spodoptera SF9), animal cells (for example, CHO, COS, HeLa, C127, 3T3, BHK, HEK293, and Bowes melanoma cell), and plant cells. The vectors can be introduced into host cells by known methods, for example, methods such as a calcium-phosphate precipitation method, electroporation (Current protocols in Molecular Biology, eds. Ausubel et al. (1987) John Wiley & Sons, Section 9.1-9.9), lipofectamine method (GIBCO-BRL), microinjection, and such.

An appropriate secretory signal may be integrated with a protein of interest such that the expressed protein is secreted into the endoplasmic reticulum lumen, cell periplasmic space, or extracellular environment of the host cells. Those signals may be endogenous or foreign to the target protein.

The proteins of the present invention can be obtained by recovering the produced proteins from the above host cells or culture supernatants thereof. When the polypeptides of the present invention are secreted into culture media, the proteins of the present invention can be recovered by collecting the culture media. Alternatively, when the proteins of the present invention are produced within the cells, the cells are first lysed and then the proteins are recovered.

The proteins of the present invention can be recovered and purified from cultures of recombinant cells by using known methods, including ammonium sulfate or ethanol precipitation, acid extraction, anion or cation exchange chromatography, phosphocellulose chromatography, hydrophobic interaction chromatography, affinity chromatography, hydroxyapatite chromatography, and lectin chromatography.

### Examples

Herein below, the present invention will be specifically described with reference to Examples, but it is not to be construed as being limited thereto.

### (1) Experimental materials and assay methods used

### (a) Construction of a reporter vector comprising HRE

A fragment comprising two repeats of the 5'-RCGTG-3' sequence, which is a hypoxia responsive element (HRE), was prepared and integrated into the multi-cloning site of plasmid pGL3-promoter vector (Promega) to construct a pHRE-Luc vector. Then, the luciferase gene was excised from pHRE-Luc at the *Hin*dIII*-Xba*I site. The EGFP gene excised from pEGFP-N1 vector (clontech) at the *Hin*dIII*-ba*I site was inserted into the above site to construct a pHRE-N1 vector. Meanwhile, CD4Δ gene was excised from pMACS4.1 at the *Eco*RI*-Hin*dIII site, and then inserted into pcDNA3.1(-) vector (Invitrogen) between the *Eco*RI*-Hin*dIII site to construct a pcDNA3.1-CD4Δ vector. Furthermore, pcDNA3.1-CD4Δ vector was digested at *Nur*I*-Pvu*I site and a fragment comprising CMV promoter, EGFP-N1, and dBGH poly(A) was obtained by partial digestion with *Pvu*II. The fragment was inserted into the pHRE-N1 vector to construct pHRE-N1-CMV-CD4Δ vector (see Fig. 1).

### (b) Introduction of genes into culture cells

HEK293 cells were cultured using D-MEM supplemented with 10% FBS (Invitrogen). Gene transfer was carried out by electroporation using Gene Pulser II (Bio-Rad); then 0.3 ml of cells at a concentration of 1 x 10⁷ cells/ml was combined with a 1:10 ratio of pHRE-N1-CMV-CD4A vector (0.5 µg) and siRNA expression vector library (5 µg). Electroporation was performed at 0.22 kV voltage and 950 µF capacitance. The cells introduced with the genes were cultured in collagen-coated type-I6-well plates (150 µl/well) (Ashahi Techno Glass) under 5% CO₂ for 48 hours.

### (c) Selection of cells

The pHRE-N1-CMV-CD4Δ vector and siRNA expression vector library were introduced into HEK293 cells by electroporation. The cells were harvested after 48 hours, and then stained with anti-human CD4 (Fujisawa Pharmaceutical Co., Ltd). About 10⁵ positive cells with an increased expression level of EGFP were collected with FACSCalibur (Becton Dickinson).

### (d) Recovery of siRNA expression vector from culture cells

Plasmids were recovered from the harvested cells using QIAamp DNA/Blood Kit (QIAGEN).

### (e) Amplification of hairpin from the recovered plasmid

A hairpin was amplified by PCR from the recovered plasmid. The primers used were: M13-47 (5'-CGCCAGGGTTTTCCCAGTCACGAC-3'/ SEQ ID NO: 225) and M13-RV (5'-agcggataacaatttcacacagg-3/ SEQ ID NO: 226). The enzyme used was 9° Nm DNA Polymerase (NEB). The PCR products were treated with EcoRI and *Sph*I and subjected to 3.5 % PAGE. The hairpin fragment was excised, eluted from the gel using Elution buffer (0.5 M AcONH₄, 10 mM AcOMg, 1 mM EDTA, and 0.1 % SDS) to be purified. The purified hairpin was ligated with *Eco*RI/*Sph*I-treated ptRNA/SS using Rapid DNA Ligation Kit (Roche).

### (f) Introduction of genes into E. coli

The ligation product was mixed with ElectroMAX DH10B Cells (Invitrogen). Gene transfer was carried out by electroporation using GENE PULSER II (BIO-RAD) in which the electroporation conditions were 2.0 kV of voltage, 25 µF of capacitance, and 200 Ω of resistance. *E. coli* cells introduced with the genes were plated onto two LB (+Amp) plates (type2 square plates), and incubated at 37°C overnight. The following morning, some were stored as glycerol stocks, and plasmids were recovered from the remainder using a HiSpeed Plasmid Midi Kit (QIAGEN).

### (g) Sequencing

Diluted glycerol stocks were plated onto LB media (Amp) and incubated at 37°C overnight. The colonies were selected and cultured, and then the plasmids were recovered using Multiscreen FB (Millipore). Since it is difficult to sequence the hairpin as is, sequencing was performed after cleaving the loop with *Bam*HI (NEB). Sequencing was carried out using a DYEnamic ET Terminator Cycle sequencing kit (Amersham Bioscience). Sense and antisense sequences were determined using the M13-47 and M13-RV primers, respectively. The NCBI BLAST program was used to search the obtained sequences for genes from standard databases.

### (2) Results of preparing a reporter vector comprising HRE

An evaluation vector (comprising HRE) and a cDNA siRNA library were co-transfected. Thus, as well as fusing an HRE reporter gene with the evaluation vector, it is also necessary to incorporate a constitutively expressed gene, which is used to confirm whether the plasmid is successfully introduced into cells. Thus, the evaluation vector (pHRE-N1-CMV-CD4Δ vector) was constructed using EGFP-N1 as an HRE reporter gene, and a CMV promotor into which CD4Δ was incorporated was used to confirm whether the plasmid was introduced into the cells (see Fig. 1).

### (3) Results of screening the cDNA siRNA library for target genes using pHRE-N1-CMV-CD4Δ vector

From the siRNA library the present inventors aimed to obtain siRNAs for genes that suppress the expression of genes downstream of HRE under normal oxygen conditions. Then, HEK293 cells were co-transfected with the siRNA expression vector library and pHRE-N1-CMV-CD4Δ vector by electroporation, and cells with elevated EGFP expression as compared with the control were collected by sorting. Plasmids were recovered from the collected cells, and the hairpin fragments were amplified by PCR and cloned. The cloned plasmids were re-introduced into cultured cells and cells with elevated EGFP expression were collected by sorting. The hairpin was amplified by PCR and cloned.

### (4) Results of screening evaluation

576 clones were selected by screening. The clones were cultured, and then the plasmids were recovered and sequenced. The obtained sequences were searched using the NCBI BLAST program, and VHL (von-Hippel Lindau syndrome) was found as a result (SEQ ID NOs: 1 to 33). At normal oxygen concentrations VHL has been reported to bind to HIF1α which binds to HRE, and by degrading HIF1α it indirectly suppresses the expression of angiogenesis-enhancing genes downstream of HRE (Maxwell PH., et al. Nature 399: 271-275, 1999). The fact that this screening has preferentially detected VHL suggests that the screening system works very effectively.

### Industrial Applicability

The present invention provides libraries of expression vectors for stem-loop RNA molecules that enable comprehensive screening for gene function, and also provides comprehensive methods of screening for drug target molecules using these libraries. These methods also enable the identification of drug target molecules whose suppressed expression is expected to produce elevated effects. In addition, the present invention suggests the possibility that where altering the expression of target genes is expected to have therapeutic effects, factors regulating target gene expression under normal conditions can be identified, and thus gene expression can be controlled by suppressing the expression of these factors. When such factors suppress target gene expression, suppressing the expression of the factors can enhance target gene expression. Alternatively, when the factors enhance target gene expression, suppressing the expression of the factors can suppress target gene expression. Specifically, the factors identified were those whose repressed expression indirectly enhanced the expression of angiogenesis-enhancing genes downstream of HRE. Such factors are novel drug targets, and at the same time siRNA molecules that suppress the expression of the factors are applicable to pharmaceuticals having novel drug effects. In addition, expression vectors for stem-loop RNA molecules that suppress the expression of the factors would be applicable to gene therapy.

## Claims

1. A DNA for functional-gene screening that comprehensively screens for a factor that regulates the expression of a target gene, and which encodes a stem-loop RNA molecule that has an RNAi effect in a cell and is structured such that a promoter is operatively linked to a DNA encoding an RNA corresponding to a region arbitrarily selected from a known nucleotide sequence and a sequence complementary to the DNA, which are linked in opposite direction *via* a spacer region.

2. The DNA of claim 1, wherein the promoter is a PolIII promoter.

3. The DNA of claim 2, wherein the promoter is a tRNA promoter.

4. The DNA of claim 3, wherein the tRNA promoter is a tRNA^{VAL} promoter.

5. The DNA of claim 1, wherein the length of the region arbitrarily selected from the known nucleotide sequence is 20 bp to 40 bp:

6. The DNA of claim 1, wherein the length of the spacer region is 1 bp to 20 bp.

7. The DNA of claim 1, wherein the length of the stem region of the expressed stem-loop RNA molecule is 20 bp to 40 bp.

8. The DNA of claim 1, wherein the target gene isa secretory protein.

9. The DNA of claim 1, wherein the factor that regulates the expression of the target gene is a protein.

10. A stem-loop RNA molecule that is a transcript of the DNA of any of claims 1 to 9 and which produces an RNAi effect in a cell.

11. An siRNA molecule generated from a transcript of the DNA of any of claims 1 to 9 and which produces an RNAi effect in a cell.

12. A synthetic siRNA molecule that has an RNAi effect in a cell and whose sequence corresponds to that of an siRNA generated in a cell from the transcript of the DNA in any of claims 1 to 9.

13. A vector carrying the DNA of any of claims 1 to 9.

14. A cell retaining the vector of claim 13 or the DNA of any of claims 1 to 9.

15. The cell of claim 14 that is a mammalian cell.

16. A composition comprising the vector of claim 13 or the DNA of any of claims 1 to 9.

17. A method for producing a cell for functional-gene screening, which comprises the steps of introducing the vector of claim 13 or the DNA of any of claims 1 to 9 into a cell, and selecting a cell introduced with the vector.

18. A method of functional-gene screening that comprehensively screens for a factor that regulates the expression of a target gene, which comprises the steps of:
(a) introducing the vector of claim 13 into a cell;
(b) selecting a cell introduced with the vector;
(c) analyzing the phenotype of the selected cell; and
(d) screening for a functional gene based on the sequence of the stem-loop RNA in the vector sequence of the cell found by analyzing the phenotype to have an altered phenotype.

19. The method of functional-gene screening of claim 18, which further comprises the step of introducing a cell with a vector comprising a DNA structured such that a promoter is operatively linked to a DNA encoding the regulatory region for expression of a target gene and a DNA encoding a marker gene that alters the phenotype of the cell.

20. The method of functional-gene screening of claim 18 or 19, which identifies a gene that indirectly enhances or suppresses the expression of a target gene.

21. A method for confirming the function of the identified functional gene of claim 20, which comprises the step of introducing the vector of claim 13 or the DNA of any of claims 1 to 9 into a cell or experimental animal.

22. A method for confirming the function of the identified functional gene of claim 20, which comprises the step of introducing the synthetic siRNA molecule of claim 12 into a cell or experimental animal.

23. The method of functional-gene screening of claim 18 or 19, wherein the target gene is a gene associated with hypoxic stimulation.

24. The method of functional-gene screening of claim 18 or 19, wherein the target gene is a gene whose expression is enhanced by HIF.

25. The method of functional-gene screening of claim 19, wherein the regulatory region for expression of the target gene comprises HRE.

26. A polynucleotide comprising the nucleotide sequence of any of SEQ ID NOs: 1 to 224.

27. A polynucleotide comprising a 19 bp or more consecutive nucleotide sequence region, wherein the nucleotide sequence is arbitrarily selected from any of SEQ ID NOs: 1 to 224.

28. A polynucleotide encoding a protein associated with hypoxic stimulation, which comprises the nucleotide sequence region of claim 27.

29. A protein associated with hypoxic stimulation, which is encoded by the polynucleotide of claim 28.

30. A vector comprising the polynucleotide of any of claims 26 to 28.

31. A host cell retaining the vector of claim 30 or the polynucleotide of any of claims 26 to 28.

32. A method for producing the protein of claim 29, which comprises the step of culturing the host cell of claim 31 and recovering the produced protein from the host cell or culture supernatant thereof.

33. An siRNA that cleaves the transcript of the polynucleotide of claim 27.
